(19) Europäisches Patentamt
European Patent Office
Office européen des brevets

(11) EP 3 361 250 B1

(12) EUROPEAN PATENT SPECIFICATION

(45) Date of publication and mention of the grant of the patent:
**21.07.2021 Bulletin 2021/29**

(21) Application number: **16849065.4**

(22) Date of filing: **26.09.2016**

(51) Int Cl.:
*A61K 31/165* (2006.01) *G01N 33/50* (2006.01)
*G01N 33/94* (2006.01) *A61K 31/16* (2006.01)
*A61K 31/122* (2006.01) *A61P 9/02* (2006.01)
*A61P 21/00* (2006.01) *A61P 5/48* (2006.01)

(86) International application number:
**PCT/KR2016/010781**

(87) International publication number:
**WO 2017/052340 (30.03.2017 Gazette 2017/13)**

# (54) MIDODRINE FOR INDUCING EXERCISE-LIKE EFFECTS

MIDODRINE ZUR INDUKTION VON ÜBUNGSÄHNLICHEN EFFEKTEN

MIDODRINE POUR L'INDUCTION D'EFFETS DE TYPE EXERCICE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.09.2015 KR 20150136009**

(43) Date of publication of application:
**15.08.2018 Bulletin 2018/33**

(73) Proprietor: **Cellvertics Co., Ltd.**
**Seoul 06621 (KR)**

(72) Inventors:
- **SEO, Hong Seog**
  **Seoul 04322 (KR)**
- **KIM, Eung Ju**
  **Seoul 08016 (KR)**
- **LEE, Yong Jik**
  **Incheon 21553 (KR)**
- **KIM, Hyeon Soo**
  **Seoul 01716 (KR)**

(74) Representative: **Pföstl, Andreas**
**Schwarz & Partner Patentanwälte**
**Wipplingerstraße 30**
**1010 Wien (AT)**

(56) References cited:
**EP-A1- 3 124 019** **WO-A2-2008/003093**
**WO-A2-2008/003093** **KR-A- 20130 065 133**
**US-A1- 2004 063 719**

- **SCHRAGE WILLIAM G; EISENACH JOHN H; DINENNO FRANK A; ROBERTS SHELLY K; JOHNSON CHRISTOPHER P; SANDRONI PAOLA; LOW PHILIP A; JOYNER: "Effects of midodrine on exercise-induced hypotension and blood pressure recovery in autonomic failure", JOURNAL OF APPLIED PHYSIOLOGY., vol. 97, no. 5, 23 July 2004 (2004-07-23), pages 1978-1984, XP055226681, US ISSN: 8750-7587, DOI: 10.1152/japplphysiol.00547.2004**
- **SCHRAGE WILLIAM G ET AL: "Midodrine reduces post-exercise calf vascular conductance in Pure Autonomic Failure patients", FASEB JOURNAL, vol. 18, no. 4-5, 2004, XP9512911, & FASEB MEETING ON EXPERIMENTAL BIOLOGY: TRANSLATING THE GENOME; WASHINGTON, DISTRICT OF COLUMBIA, USA; APRIL 17-21, 2004 ISSN: 0892-6638**
- **BRÄNDLE J ET AL: "[Investigations of the effect of midodrine on carbohydrate and fat metabolism with particular reference to the diabetic metabolic state (author's transl)].", WIENER KLINISCHE WOCHENSCHRIFT 04 MAR 1977, vol. 89, no. 5, 4 March 1977 (1977-03-04), pages 164-167, XP9512916, ISSN: 0043-5325**

EP 3 361 250 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- EISENACH JOHN H ET AL: "Midodrine improves exercise-induced hypotension in pure autonomic failure", MEDICINE & SCIENCE IN SPORTS & EXERCISE, vol. 36, no. 5, Suppl. S, May 2004 (2004-05), page S157, XP9512909, & 51ST ANNUAL MEETING OF THE AMERICAN-COLLEGE-OF-SPORTS-MEDICINE; INDIANAPOLIS, IN, USA; JUNE 02 -05, 2004 ISSN: 0195-9131
- XU, MING ET AL.: 'al-adrenergic Receptors Activate AMP-activated Protein Kinase in Rat Hearts' ACTA PHYSIOLOGICA SINICA vol. 59, no. 2, 25 April 2007, pages 175 - 182, XP055371238
- RUDERMAN, NEIL ET AL.: 'AMP Kinase and Malonyl-CoA: Targets for Therapy of the Metabolic Syndrome' NATURE REVIEWS DRUG DISCOVERY vol. 3, no. 4, April 2004, pages 340 - 351, XP055371245

## Description

CROSS-REFERENCE TO RELATED APPLICATION

This application claims priority to and the benefit of Korean Patent Applications Nos. 2015-0136009, 2016-0122779 and 2016-0122780 filed on September 25, 2015, September 26, 2016, and September 26, 2016, respectively.

### BACKGROUND

#### 1. Field of the Invention

The present disclosure relates to a pharmaceutical composition for inducing an exercise mimetic effect, which contains an $\alpha_1$-adrenergic receptor agonist as an active ingredient, and a method for screening a drug for inducing an exercise mimetic effect using the $\alpha_1$-adrenergic receptor agonist.

#### 2. Description of Related Art

Exercise training improves exercise tolerance by activating the remodeling program causing change in the phenotype of skeletal muscle and moderate exercise is effective in improving the pathological conditions of metabolic diseases, heart diseases, etc.

Especially, AMPK (AMP-activated protein kinase), PPAR-$\delta$ (peroxisome proliferator-activated receptor-$\delta$) and PGC-1$\alpha$ (peroxisome proliferator-activated receptor gamma coactivator-1$\alpha$) are known as important factors involved in the change in the phenotype of skeletal muscle for exercise tolerance.

AMPK, which is a heterotrimeric complex consisting of a, $\beta$ and $\gamma$ subunits, is activated by phosphorylation during muscle contraction and exercise by LKB1 and CaMKK ($Ca^{2+}$/calmodulin-dependent kinase kinase) and acts as a major regulator in the cellular/organ metabolisms for glucose homeostasis, appetite and exercise physiology. PPAR-$\delta$ plays a critical role in the transcriptional regulation of skeletal muscle metabolism. PGC-1$\alpha$ is involved in energy metabolism as a regulator of mitochondrial biosynthesis and function. It is a transcriptional coactivator that regulates the genes activated by the exercise tolerance of skeletal muscle.

AMPK is known to be capable of targeting several transcriptional programs at the same time which are regulated by substrates such as PPAR-$\delta$ and PGC-1$\alpha$ and induce genetic effects similar to that of exercise. In this regard, it is reported that drugs targeting the AMPK/PPAP$\delta$ signaling pathway can become a new pharmaceutical strategy for reprogramming muscles (Cell 2008, vol. 134, pp. 405-415).

In addition, it is reported that some drugs facilitating AMPK activation such as A-769662, metformin, 5-aminoimidazole-4-carboxamide-1-$\beta$-D-ribofuranoside (AICAR) and resveratrol exert therapeutic effects for heart failure. The effect of protecting the heart by AMPK can be achieved through important functions such as decreased production of reactive oxygen species in the cytoplasm, inhibition of angiotensin II activity, phosphorylation of cardiac troponin I, activation of PGC-1$\alpha$, regulation of eNOS-NAD(P)H oxidase expression, regulation of estrogen-related receptors, regulation of energy balancing and intracardiac signaling, etc. The activation of AMPK can improve the cardiac muscle function directly or indirectly.

As described, because AMPK activation (phosphorylation) and the factors related thereto (PPAR-$\delta$ and PGC-1$\alpha$) induce exercise mimetic effects such as glucose uptake in skeletal muscle, maintenance of homeostasis in energy metabolism, enhancement of cardiac function, etc., there have been consistent demands on drugs that activate AMPK as a target for metabolic diseases, cardiovascular diseases, inflammatory diseases, etc. that can be treated by the exercise mimetic effect.

EP 3 124 019 discloses the use of midodrine in the treatment of diseases like obstructive cardiopathy

KR 2013 0065133 A discloses the activity of midodrine as a- receptor agonist in influencing the blood pressure by inducing rising, which is caused through the activation of the alpha-adrenergic receptor of the vein or the arterial vessel.

Schrage WG et al. (J Appl Physiol 97 (2004): 1978-1984) suggest using midodrine to limit the fall in arterial pressure during exercise in people suffering from pure autonomic failure. They conclude that midodrine improves blood pressure during exercise.

Schrage WG et al. (FASEB J 18(2004)) discloses the improvement of the recovery from exercise-induced hypotension by administering midodrine.

Brändle J et al. (Wiener Klinische Wochenschrift 89(1977): 164-167) describes the effects on carbohydrate and fat metabolism in healthy subjects and patients with disturbed glucose tolerance treated with midodrine.

In Eisenach J et al. (Med Sci Sports and Exercise 36 (Suppl) (2004): 157) the increase of the vascular resistance during exercise and blood pressure recovery after exercise by administering midodrine is disclosed.

## SUMMARY OF THE INVENTION

The inventors of the present disclosure have found out that hypertension, metabolic diseases such as obesity and diabetes, etc., heart diseases and inflammatory diseases can be improved by activating the $\alpha_1$-adrenergic receptor and have researched its mechanism of action. As a result, they have found out that the activation of the $\alpha_1$-adrenergic receptor increases the expression of activated AMPK, PPAR-$\delta$ and PGC-1$\alpha$ and, through this, can induce an exercise mimetic effect in multiple organs such as skeletal muscle, cardiac muscle, liver, etc. and have completed the present disclosure.

The present invention is directed to providing a pharmaceutical composition for use in the treatment of a disease requiring the activation of an AMP-activated protein kinase (AMPK), comprising an $\alpha_1$-adrenergic receptor agonist or a pharmaceutically acceptable salt thereof as an active ingredient selected from the group consisting of midodrine, 2-amino-N-(2-(2,5-dimethoxyphenyl)-2-hydroxyethyl)-3-phenylpropanamide, 2-amino-N-(2-(2,5-dimethoxyphenyl)-2-hydroxyethyl)propanamide, 2-amino-N-(2-(3,5-dimethoxyphenyl)-2-hydroxyethyl)acetamide hydrochloride, 2-amino-N-(2-(5-ethyl-2-methoxyphenyl)-2-hydroxyethyl)acetamide hydrochloride and 2-amino-N-(2-hydroxy-2-phenylethyl)acetamide hydrochloride, wherein the disease is selected from hypertension, hyperlipidemia, diabetes, obesity, arteriosclerosis, fatty liver and inflammatory disease.

The present disclosure is also directed to providing a method for screening a drug for inducing an exercise mimetic effect using the $\alpha_1$-adrenergic receptor agonist.

The present disclosure relates to a pharmaceutical composition for inducing an exercise memetic effect, which contains an $\alpha_1$-adrenergic receptor agonist or a pharmaceutically acceptable salt thereof as an active ingredient.

The present disclosure also relates to a method for inducing an exercise mimetic effect, which includes a step of administering an $\alpha_1$-adrenergic receptor agonist or a pharmaceutically acceptable salt thereof to a subject.

The present disclosure also relates to a use of an $\alpha_1$-adrenergic receptor agonist or a pharmaceutically acceptable salt thereof for inducing an exercise mimetic effect.

In a specific exemplary embodiment of the present disclosure, the $\alpha_1$-adrenergic receptor agonist induces the activation of AMPK.

In another specific exemplary embodiment of the present disclosure, the $\alpha_1$-adrenergic receptor agonist induces the expression of PPAR-$\delta$ or PGC-1a.

In another specific exemplary embodiment of the present disclosure, the $\alpha_1$-adrenergic receptor agonist is midodrine.

In another specific exemplary embodiment of the present disclosure, the exercise mimetic effect is an effect of treating a disease requiring the activation of AMPK.

The present invention also relates to a method for screening a drug for inducing an exercise mimetic effect using an $\alpha_1$-adrenergic receptor agonist.

The screening method includes: (a) a step of treating a cell with an $\alpha_1$-adrenergic receptor agonist *in vitro;* and (b) a step of measuring the expression of phosphorylated AMPK (AMP-activated protein kinase), PPAR-$\delta$ (peroxisome proliferator-activated receptor-$\delta$) or PGC-1$\alpha$ (peroxisome proliferator-activated receptor gamma coactivator-1$\alpha$).

In another exemplary embodiment of the present disclosure, the screening method further comprises (c) a step of selecting the agonist as a drug when the expression of phosphorylated AMPK, PPAR-$\delta$ or PGC-1$\alpha$ is increased as compared to a group not treated with the agonist.

In another exemplary embodiment of the present disclosure, in the step (b), the expression is measured by western blotting, antigen immunoprecipitation, ELISA, mass spectrometry, RT-PCR, competitive RT-PCR, real-time RT-PCR, RPA (RNase protection assay) or northern blotting.

In another exemplary embodiment of the present disclosure, in the step (a), the cell is the cell of a tissue selected from a group including skeletal muscle, cardiac muscle, liver, fat and pancreas.

In another exemplary embodiment of the present disclosure, the exercise mimetic effect is an effect of treating a disease selected from a group including a metabolic disease, a cardiovascular disease and an inflammatory disease.

In another exemplary embodiment of the present disclosure, the metabolic disease is a disease selected from a group including hypertension, hyperlipidemia, diabetes, obesity, arteriosclerosis and fatty liver.

An $\alpha_1$-adrenergic receptor agonist of the present disclosure increases the expression of p-AMPK, PPAR$\delta$ and PGC-1$\alpha$, which play key roles in maintaining and regulating energy metabolic activity *in vivo,* thereby increasing glucose uptake into skeletal muscle cells, suppressing adipocyte differentiation and lipid accumulation, reducing abdominal fat and body weight as well as regulating mitochondrial metabolic disturbances and suppressing inflammatory responses. Accordingly, the $\alpha_1$-adrenergic receptor agonist can be usefully used to prevent and treat diseases requiring AMPK activation (metabolic diseases, cardiovascular diseases, inflammatory disease, etc.).

A composition of the present disclosure can be used directly for clinical application because a drug free from side effects and proven safety is used (drug repositioning).

And, according to a method of the present disclosure, a drug inducing an exercise mimetic effect can be

screened conveniently with high precision *in vitro.*

## BRIEF DESCRIPTION OF THE DRAWINGS

FIG. 1 (A) and (B) show a western blot result showing that the expression of activated (phosphorylated) AMPK (p-AMPK) and PPARδ proteins is increased in skeletal muscle, cardiac muscle and liver cells when the $\alpha_1$-adrenergic receptor ($\alpha_1$-AR) is activated by midodrine.

FIG. 2a shows a result of investigating the expression of $\alpha_1$-AR protein in the skeletal muscles of a 4-week-old basal control group rat (I), a midodrine-administered rat (II), an atenolol-administered rat (III) and an 8-week-old unadministered control group rat (IV) by western blotting and FIGS. 2b-2d show a result of investigating the expression of AMPK, PPARδ and PGC1α proteins in the cardiac muscles, skeletal muscles and livers, respectively, of the rat groups by western blotting.

FIGS. 3a and 3b show a northern blot result of investigating the mRNA expression level of genes associated with vasoconstriction, nitric oxide production, oxidative stress and inflammation, respectively, in the aortic tissues (FIG. 3a) and the cardiac muscle tissues (FIG. 3b) of a 4-week-old basal rat (I), a midodrine-administered rat (II), an atenolol-administered rat (III) and an 8-week-old unadministered control group rat (IV).

FIG. 4a shows a result of measuring the enzyme activity of SDH (succinate dehydrogenase) in the skeletal muscles of a 4-week-old basal rat (I), a midodrine-administered rat (II), an atenolol-administered rat (III) and an 8-week-old unadministered control group rat (IV) and FIG. 4b shows an immunohistochemical staining result of cytochrome c oxidase for the skeletal muscle tissues of the above groups.

FIG. 5 shows a result of measuring the ATP level in the cardiac muscles, skeletal muscles and livers of a 4-week-old basal rat (I), a midodrine-administered rat (II), an atenolol-administered rat (III) and an 8-week-old unadministered control group rat (IV) by ELISA.

FIG. 6 shows a result of investigating the effect of midodrine on glucose uptake by insulin in mouse skeletal muscle cells (C2C12 cells).

FIG. 7a shows that fat synthesis and accumulation are decreased in differentiated adipocytes after administration of midodrine and the effect is suppressed after administration of a PPARδ antagonist and FIG. 7b shows a result of investigating the mechanism of the effect of midodrine on the suppression of fat synthesis and accumulation in the adipocytes through expression of PPARδ, p-AMPK and PGC-1α proteins by western blotting.

FIG. 8 shows a result of investigating the effect of midodrine on body weight and abdominal fat weight.

FIG. 9 (A) and (B) show a result of investigating the effect of midodrine on the expression of the mannose receptor (MR) and hexokinase II in inflammation-associated macrophages. FIG. 9 (C) shows an immunohistochemical staining result for investigating the mannose receptor expression of macrophages localized in the subcapsular portion of the spleens in a 4-week-old basal rat (I), a midodrine-administered rat (II), an atenolol-administered rat (III) and an 8-week-old unadministered control group rat (IV). It can be seen that the expression of the mannose receptor is significantly increased in the midodrine-administered group (II) 23010-WOEP EP 16849065.4 as compared to other animal groups.

FIG. 10 shows a western blot result showing that the expression of p-AMPK (A) and PPAR-δ (B) proteins in mouse skeletal muscle cells (C2C12) when the $\alpha_1$-adrenergic receptor ($\alpha_1$-AR) is activated by Compound 5.

FIG. 11 shows a western blot result showing that the expression of p-AMPK (A) and PPAR-δ (B) proteins in mouse skeletal muscle cells (C2C12) when the $\alpha_1$-adrenergic receptor ($\alpha_1$-AR) is activated by Compound 7.

FIG. 12 shows a western blot result showing that the expression of p-AMPK (A), PPAR-δ (B) and PGC-1α (C) proteins in mouse skeletal muscle cells (C2C12) when the $\alpha_1$-adrenergic receptor ($\alpha_1$-AR) is activated by Compound 8.

FIG. 13 shows a western blot result showing that the expression of p-AMPK (A), PPAR-δ (B) and PGC-1α (C) proteins in mouse skeletal muscle cells (C2C12) when the $\alpha_1$-adrenergic receptor ($\alpha_1$-AR) is activated by Compound 9.

FIG. 14 shows a western blot result showing that the expression of p-AMPK (A), PPAR-δ (B) and PGC-1α (C) proteins in mouse skeletal muscle cells (C2C12) when the $\alpha_1$-adrenergic receptor ($\alpha_1$-AR) is activated by Compound 10.

## DETAILED DESCRIPTION OF THE INVENTION

The present disclosure provides a pharmaceutical composition for use in the treatment of a disease requiring the activation of an AMP-activated protein kinase (AMPK), which contains an $\alpha_1$-adrenergic receptor agonist or a pharmaceutically acceptable salt thereof as an active ingredient.

The present disclosure has been devised based on the finding that the $\alpha_1$-AR agonist can exert an exercise mimetic effect in multiple organs such as skeletal muscle, cardiac muscle, liver, etc. through AMPK activation in addition to its intrinsic effect of improved cardiac muscle contraction through stimulation of $\alpha_1$-AR.

The $\alpha_1$-adrenergic receptor agonist is a substance acting on and activating the $\alpha_1$-adrenergic receptor. There are three subtypes $\alpha_1$, $\alpha_2$ and $\beta$ of the adrenergic receptor. In the present disclosure, any known compound that activates the $\alpha_1$-type receptor can be used without limitation. Examples may include midodrine, analogues thereof or pharmaceutically acceptable salts thereof.

The midodrine compound is marketed under the brand names Amatine, ProAmatine, Gutron, etc. Its IUPAC name is (R/S)-N-[2-(2,5-dimethoxyphenyl)-2-hydroxyethyl]glycinamide and is represented by Chemical Formula I.

[Chemical Formula 1]

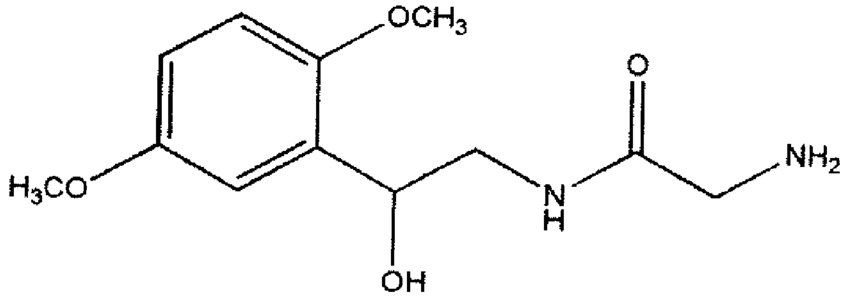

Midodrine is a prodrug which is changed into a target compound *in vivo* after being administered. After the administration, it is changed into the active metabolite, desglymidodrine, which activates the $\alpha_1$-adrenergic receptor, thereby inducing AMPK activation and PPAR-$\delta$ or PGC-1a expression and finally inducing an exercise mimetic effect.

The compound represented by Chemical Formula 1 may form "a pharmaceutically acceptable salt". A suitable pharmaceutically acceptable salt is one commonly used in the art to which the present disclosure belongs, such as an acid addition salt, and is not specially limited. Specifically, the acid that may be used in the pharmaceutically acceptable acid addition salt may be, for example, an inorganic acid such as hydrochloric acid, sulfuric acid, nitric acid, phosphoric acid, perchloric acid or bromic acid or an organic acid such as acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid or glutamic acid. An organic base that may be used to prepare an organic base addition salt may be, for example, tris(hydroxymethyl)methylamine, dicyclohexylamine, etc. An amino acid that may be used to prepare an amino acid addition base may be a naturally occurring amino acid such as alanine, glycine, etc.

Also, in the present disclosure, the midodrine "analogue" is not specially limited as long as it is a compound having a structure similar to that of midodrine and exhibiting an effect comparable to that of midodrine. Examples may include the following Compounds 5, 7, 8, 9 and 10.

Compound 5: 2-amino-N-(2-(2,5-dimethoxyphenyl)-2-hydroxyethyl)-3-phenylpropanamide

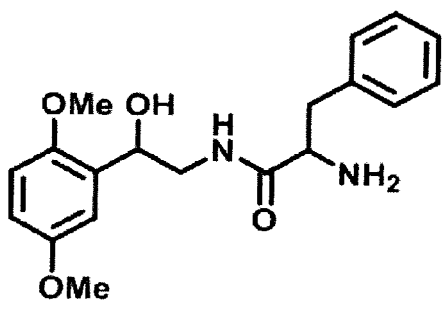

Compound 7: 2-amino-N-(2-(2,5-dimethoxyphenyl)-2-hydroxyethyl)propanamide

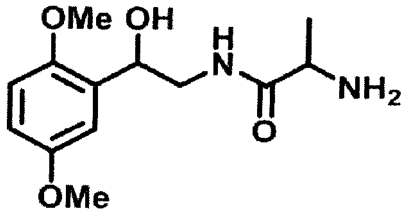

Compound 8: 2-amino-N-(2-(3,5-dimethoxyphenyl)-2-hydroxyethyl)acetamide hydrochloride

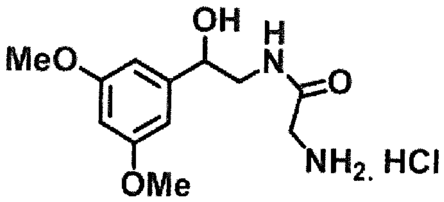

Compound 9: 2-amino-N-(2-(5-ethyl-2-methoxyphenyl)-2-hydroxyethyl)acetamide hydrochloride

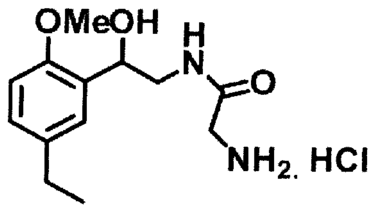

Compound 10: 2-amino-N-(2-hydroxy-2-phenylethyl)acetamide hydrochloride

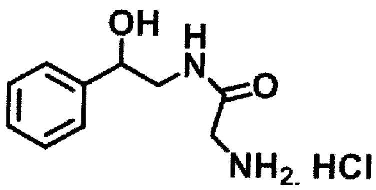

In the present disclosure, the $\alpha_1$-adrenergic receptor agonist may achieve an exercise mimetic effect by inducing AMPK activation and PPAR-$\delta$ or PGC-1$\alpha$ expression.

AMPK acts as an energy sensor which senses energy state *in vivo* and maintains it at a constant level. When the energy in a cell is decreased due to, for example, metabolic stress or exercise, i.e., when the AMP/ATP ratio is increased due to depletion of ATP, AMPK is activated and facilitates the processes consuming ATP (e.g., fatty acid oxidation or glycolysis). AMPK activation induces metabolically important results in major target organs (liver, muscles, fats or pancreas). It is known to inhibit fatty acid and cholesterol synthesis and facilitate fatty acid oxidation in the liver, facilitate fatty acid oxidation and glucose uptake in skeletal muscles, inhibit fat synthesis in adipocytes and facilitate insulin secretion in pancreatic $\beta$ cells.

PPAR-$\delta$ is known to facilitate catabolic energy metabolism in cells by regulating AMPK and play an essential role in maintaining metabolic balance (homeostasis) such as anti-inflammation, inhibition of insulin resistance, etc.

PGC-1a is a major regulator of mitochondrial biogenesis. It is known that its expression is induced by severe metabolic changes such as exercise, starvation, cold, etc. and is regulated by AMPK, PPAR-δ, NAD-dependent deacetylase sirtuin-1 (SIRT1), etc.

In the present disclosure, the exercise mimetic effect means a physiological effect exerted during exercise such as improvement in cardiac function (increase in contractile force), increase in the insulin sensitivity and oxidative phosphorylation of muscles, decrease in cholesterol, decrease in fat accumulation and body weight, decrease in blood inflammation, etc., although not being specially limited thereto.

In the present disclosure, the disease requiring AMPK activation refers to various diseases that can be cause by deactivation of AMPK without special limitation. For example, it may be a metabolic disease, a cardiovascular disease, an inflammatory disease, etc.

In the present disclosure, the "metabolic disease" refers to a disease caused by abnormal metabolism of glucose, fats, proteins, etc. Examples may include hyperlipidemia, diabetes, obesity, arteriosclerosis, fatty liver, etc.

In the present disclosure, the "pharmaceutical composition" may further include an existing therapeutically active ingredient, an adjuvant, a pharmaceutically acceptable carrier, etc. The pharmaceutically acceptable carrier includes a saline, sterilized water, a Ringer's solution, a buffered saline, a dextrose solution, a maltodextrin solution, glycerol, ethanol, etc.

The composition may be formulated as an oral formulation such as a powder, a granule, a tablet, a capsule, a suspension, an emulsion, a syrup, an aerosol, etc., a formulation for external application, a suppository or a sterilized injectable solution according to commonly employed methods.

In the present disclosure, it is obvious to those skilled in the art than an "administration dosage" can be controlled variously depending on the body weight, age, sex, health condition and diet of a patient, number of administrations, administration method, excretion rate, the severity of a disease, etc.

In the present disclosure, a "subject" means a subject in need of treatment of a disease, more specifically a mammal such as human, non-human primates, mouse, rat, dog, cat, horse, cow, etc.

In the present disclosure, a "pharmaceutically effective amount" is determined by the kind and severity of a disease, the age and sex of a patient, administration time, administration route, excretion rate, treatment period, the drug(s) used in combination and other factors well known in the medical field. It can be easily determined by those skilled in the art as an amount that can achieve the maximum effect without a side effect in consideration of the above-described factors.

The composition of the present disclosure is not limited in "administration method" as long as it can reach the target tissue. For example, it may be administered orally, intraarterially, intravenously, transdermally, intranasally, transbronchially or intramuscularly. A daily administration dosage is about 0.0001-100 mg/kg, specifically 0.001-10 mg/kg, and the administration may be made once or several times a day.

In the present disclosure, it has been found out that the $\alpha_1$-AR agonist improves the cardiac function by increasing PPAR-δ and PGC-1α expression independently from activating AMPK by stimulating $\alpha_1$-AR. It is because it increases the rate of heart contraction and relaxation like exercise training.

Accordingly, it is thought that the $\alpha_1$-AR agonist directly stimulates heart contraction and indirectly improves cardiac function through exercise mimetic AMPK and PPARδ activation by stimulation of $\alpha_1$-AR in multiple organs including the heart, muscles and liver, thereby contributing to improvement in exercise tolerance *in vivo*.

The present invention discloses that stimulation of $\alpha_1$-AR leads to the exercise mimetic effect on the heart through the AMPK and PPARδ activation. Also, the present invention discloses that the exercise mimetic effect activated through increased PPARδ and PGC-1α expression with by AMPK is working to the heart independently.

Specifically, in the present disclosure, the effect of $\alpha_1$-AR stimulation on the expression of the genes involved in exercise mimetic effect on cardiac muscle cells and skeletal muscle cells was investigated, and the same effect on the cardiac and skeletal muscles and the liver *in vivo* was compared using a rat (SHR) which is an animal model for metabolic syndrome in human.

Also, the effect of $\alpha_1$-AR stimulation on the cardiac function/size of the heart, inflammatory cytokines, level of reactive oxygen species, adiponectin, ATP level, SDH (succinate dehydrogenase) activity, fat amount, angiotensin II AT-1 receptor expression, etc. was investigated.

As a result, it was elucidated that, even in the early age when the SHR rat shows consistent increase in blood pressure, stimulation of $\alpha_1$-AR by the $\alpha_1$-AR agonist induces activation and increased expression of AMPK, PPAR-δ and PGC-1a in the heart and skeletal muscles and increases heart cardiac contractility without cardiac hypertrophy or additional blood pressure increase.

In addition, it was confirmed that the $\alpha_1$-AR stimulation results in decreased level of inflammatory cytokines, ROS and cholesterol in blood, and that the ratio of PPARδ and PGC-1a activation to AMPK stimulation is higher in cardiac muscles than in skeletal muscles and liver. These results are associated exercise mimetic effect through AMPK-PPARδ activation, independent from the muscle contraction effect. Also, it is the first case showing that the enhanced cardiac muscle contraction by pharmacological $\alpha_1$-AR stimulation is contributed by the AMPK-PPARδ-PGC1α activation.

Also, in the present disclosure, it was elucidated that the $\alpha_1$-AR agonist is more effective than other drugs inducing the exercise mimetic effect (e.g., atenolol). It is because the $\alpha_1$-AR agonist exhibits the dual effect of improving cardiac contractility whereas other drugs exhibit only the exercise mimetic effect without direct cardiac contraction. In addition, whereas the other drugs inducing the exercise mimetic effect are used in combination to enhance their respective functions, the $\alpha_1$-AR agonist can exert the exercise mimetic effect in multiple organs at the same time.

Also, in the present disclosure, it was confirmed that, although the rats treated with midodrine showed increase in left ventricular ejection fraction as that of the animals treated with atenolol, there was a difference in the level of phosphorylated AMPK in the heart between the experimental groups. Although $\alpha$-1 AR provides a favorable effect for the heart, the degree of $\alpha$-1 AR stimulation is important in terms of long-term prognosis because the extremely enhanced cardiac $\alpha$-1 AR drive can induce pathological remodeling in contractility disturbance, gradual fibrosis and reactivation of protein genes of fibroblasts.

Also, in the present disclosure, it was confirmed that cardiac hypertrophy did not occur despite the $\alpha_1$-AR stimulation. It may be due to the effect of AMPK on inhibition of angiotensin II, stimulating autophagy, etc. In addition, the left ventricular mass was smaller for the midodrine-treated group than the atenolol-treated group, which can be explained with the difference in cardiac AT1 expression.

Also, in the present disclosure, it was confirmed through *in-vitro* and *in-vivo* experiments for the first time that, regardless of muscle exercise, $\alpha_1$-AR stimulation activates AMPK, PPAR-$\delta$ and PGC1$\alpha$ in skeletal muscles. It has been already reported that the expression of these exercise-related genes is one of the adaptive responses for maintaining endurance exercise.

In addition, in the present disclosure, it was confirmed that the $\alpha_1$-AR stimulation leads to decreased ROS level, increased activation of AMPK, PPAR-$\delta$ and PGC1$\alpha$ in the liver, and induced a systemic effect for metabolic/biochemical/inflammatory responses and significant decrease in total cholesterol, LDL-cholesterol and HDL-cholesterol.

Also, in the present disclosure, it was confirmed that the $\alpha_1$-AR stimulation leads to increased glucose uptake by insulin, inhibited fat differentiation into adipocytes and fat synthesis/accumulation, body weight, abdominal fat amount, and inflammation. Therefore, it can be seen that the $\alpha_1$-AR stimulation is effective in such diseases as diabetes, obesity, inflammation, etc.

Also, in the present disclosure, it was confirmed that, in addition to the $\alpha_1$-adrenergic receptor agonist midodrine, various newly synthesized compounds similar thereto can induce AMPK activation and PPAR-$\delta$ and PGC-1a expression in skeletal muscle cells by stimulating $\alpha_1$-AR, thereby inducing an exercise mimetic effect.

Hereinafter, the present disclosure will be described in detail through examples. However, the following examples are for illustrative purposes only and it will be apparent to those of ordinary skill in the art that the scope of the present disclosure is not limited by the examples.

## Examples

### Example 1: Methods

#### 1-1. Materials

The TRIzol reagent was purchased from Invitrogen (CA, USA). The Power cDNA synthesis kit and the Maxime PCR PreMix kit were purchased from iNtRON Biotechnology (Seongnam-si, Gyeonggi-do, Korea). The PREP™ protein extraction solution and a prestained protein size marker were purchased from iNtRON Biotechnology. The anti-AMPKa (a subunit) and anti-phospho-AMPKa (phosphorylated at Thr172) primary antibodies were purchased from Cell Signaling Technology, Inc. (Danvers, MA, USA). The anti-rabbit secondary antibody was purchased from Santa Cruz Biotechnology (Santa Cruz, CA, USA). The Clarity Western ECL Substrate kit was purchased from Bio-Rad (Hercules, CA, USA). An X-ray film was purchased from Agfa (Mortsel, Belgium) and the development/ fixation kit was purchased from Kodak (Rochester, NY, USA). The rat adiponectin detection ELISA kit was purchased from Abcam (Cambridge, UK).

#### 1-2. Effect of $\alpha_1$-AR stimulation on AMPK in skeletal muscle/cardiac muscle cells *in vitro*

In order to investigate the effect of $\alpha_1$-AR stimulation in on the exercise mimetic effect of muscles *in vitro,* the expression of AMPK, which is the representative protein increased during exercise, was investigated first. Then, *in-vivo* animal experiment was conducted as follows on the assumption that the expression of PPAR$\delta$ and PGC-1 a will also be increased in the skeletal muscle, cardiac muscle and liver.

### 1-3. Cell culturing and animal experiment

### Cell culturing

C2C12 cells (mouse skeletal cell line) were seeded onto a 6-well plate in a $CO_2$ incubator at 37 °C and cultured in a DMEM medium containing 10% FBS and 1% antibiotics to about 80% confluency. After replacing the medium with one containing 1% FBS, differentiation was conducted for 3 days. Then, after replacing the medium with one containing 1% FBS and then treating with a drug, experiment was conducted 24 hours later.

### Animal experiment

Spontaneously hypertensive rat (SHR) is an animal model in which hereditary hypertension is expressed. It is known that the hypertension which is the most similar to human primary hypertension is expressed. In SHR, blood pressure begins to increase around 4-6 weeks of age and hypertension is developed seriously at 8-12 weeks. SHR is widely used in studies as an animal mode of primary hypertension, especially hypertension with cardiac lesions, because it is frequently accompanied damage to the hypertensive target organs, such as cardiac hypertrophy, heart failure, renal disorder, etc.

3-week-old spontaneously hypertensive rats (SHRs) were kept under a standardized condition (21 °C, 41-62% humidity) with periodic light/dark (10/14 hour) cycles and were freely allowed to access water and laboratory feed. All the animal experiments were approved by the Korea University Institutional Animal Care and Use Committee (KUIACUC-2012-100) and were in accordance with the Guideline for the Care and Use of Laboratory Animals.

After 1 week of acclimation, the rats were divided into four groups (6 rats per group) as follows: group I (basal control group, sacrificed at 4 weeks of age), group II (administered with midodrine for 4 weeks), group III (administered with atenolol for 4 weeks), group IV (control group with no drug administration for 4 weeks). The group I and group IV were given standard feed containing no drug (K-H4 pellet, Ssniff), the group II was given the same feed together with drinking water containing midodrine (0.2 mg/kg/day) and the group III was given the same feed together with drinking water containing atenolol (1 mg/kg/day).

Blood pressure was measured with 7-day intervals by tail-cuff plethysmography using the Visitech BP2000 system (Visitech Systems Inc.).

The animals of the group I were anesthetized at 4 weeks of age while the rats of other groups were anesthetized at 8 weeks of age after being treated for 4 weeks. Blood samples were taken from the inferior vena cava and the heart, aorta, liver, skeletal muscle and visceral fat were excised cleanly and then weighed. The recovered organs were kept in a refrigerator at -80 °C or in 10% formalin for fixation.

### 1-4. Echocardiographic examination

After anesthetization by intramuscular injection of Zoletil (8 mg/kg) and xylazine (2 mg/kg), the rats were laid on their left sides and M-mode echo images were obtained. The examination was conducted using Vivid 7 (GE Medical Systems, Milwaukee, WI, USA) equipped with a 12-MHz transducer. After acquiring optimized 2-dimensional short-axis images for the left ventricle at the papillary muscle level, M-mode tracing and echocardiographic images were recorded at a speed of 100 mm/s. The wall thickness, volume and mass of the heart was measured from at least three consecutive cardiac cycles on the M-mode tracing using as suggested by the American Society for Echocardiography.

### 1-5. Measurement of blood biochemistry, inflammatory markers, reactive oxygen species, ATP and adiponectin

The concentration of total cholesterol, HDL cholesterol, LDL cholesterol and triglyceride was measured by enzymatic colorimetry (Roche Diagnostics GmbH; Mannheim, Germany). Also, serum assays for inflammatory markers were conducted using a 4-plex cytokine Milliplex panel (Millipore Corporation, Billerica, MA, USA) as recommended for cytokines (interleukin (IL)-1$\alpha$, IL-1$\beta$, IL-6, IL-4, IL-10 and TNF-$\alpha$). Acquisition was performed on a Luminex 100 platform and data analysis was carried out using the Multiplex analyzer. Reactive oxygen species and adiponectin were measured using the ELISA kit (MBS815494, Mybiosource). The measured blood adiponectin level was calibrated for the visceral fat weight (g).

### 1-6. ELISA for measurement of ATP and ROS in tissue

0.02 g of liver tissue was homogenized in 500 $\mu$L of PBS. The homogenate was centrifuged at 1500 x g (or 5000 rpm) for 15 minutes and the supernatant was subjected to measurement. After pouring 100 $\mu$L of a reference material or sample to an adequate well of an antibody-precoated microtiter plate, 10 $\mu$L of the residual solution was

added to the sample. After adding 50 μL of a conjugate to each well and mixing, the plate was covered with a lid and incubated at 37 °C for 1 hour. After adding 50 μL of substrate A or B to each well, incubation was conducted at 37 °C for 15 minutes. After adding a stop solution to each well, optical density was measured at 450 nm using a microplate reader.

**1-7. Measurement of SDH (succinate dehydrogenase) activity in skeletal muscle**

After adding 10 μL of a protease inhibitor cocktail to 1 mL of PBS per well and making the volume 500 μL, 410 μL of a culture solution prepared by mixing 1 M PBS (25 μL x 25 = 625 μL), 0.2 M sodium succinate (125 μL x 25 = 3125 μL), NBT (25 μL x 25 = 625 μL) and D.W. (235 μL x 25 = 5875 μL) was heated to 37 °C 20 minutes before conducting reaction. After adding 500 μL of PBS to 0.02 g of skeletal muscle tissue, the tissue was crushed in sort time to prevent enzymes from being broken down. After centrifugation at 13000 rpm and 4 °C for 5 minutes, the supernatant was transferred to a fresh tube. 410 μL of the culture solution was transferred to a tube in advance and then enzymatic reaction was conducted by adding 90 μL of the sample. After adding 410 μL of D.W. to another tube and then adding 90 μL of the sample, the absorbance of the diluted solution was measured. The enzymatic reaction tube and another tube were put in a water bath at 37°C, respectively, and then subjected to reaction for 30 minutes. After terminating the reaction by putting the tubes in ice, 200 μL of each solution was transferred to a 96-well plate and absorbance was measured at 550 nm. The enzyme activity of succinate dehydrogenase (SDH) was calculated from the result according to the following equation.

Enzyme activity = (absorbance of enzymatic reaction solution - absorbance of diluted enzyme solution) / protein quantity (Bradford 595 nm)

**1-8. Reverse transcription polymerase chain reaction (RT-PCR) for quantification of mRNA in aorta and heart**

Full-length RNA was extracted using the TriZol reagent according to the manufacturer's instructions. Complementary DNA was synthesized from the full-length RNA using the Power cDNA Synthesis kit and polymerase chain reaction was conducted for angiotensin II type I receptor (AT1R), endothelial nitric oxide synthase (eNOS), superoxide dismutase (SOD), gp91-phox (NADPH), tumor necrosis factor-alpha (TNF-α) and GAPDH using the PCR Premix kit.

**1-9. Western blot**

The protein content of the extract was measured by the Bradford's method. The extracted proteins (20-30 μg) were loaded on a 10% SDS-PAGE gel. Western blot assay was conducted using primary antibodies for AMPKa, phosphorylated AMPKa, PPARδ and PGC-1α. Images were obtained manually using the Kodak GBX developer and a fixative reagent.

**1-10. Statistical analysis**

All the recorded blood pressure measurements were analyzed [4- to 8-week-old three animal groups]. The blood pressure was compared by ANOVA and was regarded significant when $p < 0.05$. Continuous variables were represented by mean ± standard deviation (SD). The overall difference between four groups was analyzed by the Kruskal-Wallis test. The difference between two groups was evaluated by the Mann-Whitney U-test. p-values smaller than 0.05 were regarded statistically significant. All statistical analysis was conducted using SPSS (ver. 20.0; SPSS Inc., Chicago, IL, USA).

**Example 2: Results**

**2-1. Effect of $\alpha_1$-AR stimulation on AMPK phosphorylation in skeletal muscle/cardiac muscle cells *in vitro***

In order to investigate the effect of $\alpha_1$-AR ($\alpha_1$-adrenergic receptor) stimulation on AMPK and phosphorylated (activated) AMPK *in vitro,* rat skeletal muscle cells (L6) and mouse cardiac muscle cells (HL1) were treated with the $\alpha_1$-AR agonist midodrine.

As seen from FIG. 1 (a) and (b), AMPK was expressed in the skeletal muscle cells in response to the $\alpha_1$-AR stimulation and the expression of phosphorylated AMPK was increased, depending upon the concentration of midodrine, suggesting that the $\alpha_1$-AR stimulation is associated with AMPK activation. In addition, the expression and phosphorylation of AMPK in response to the $\alpha_1$-AR stimulation by midodrine administration were also observed for the cardiac muscle cells.

Accordingly, because midodrine induces the change (AMPK activation) in cardiac muscle that also observed on skeletal muscle in response to midodrine-in addition to the previously known improvement of cardiac muscle contractility, it can be seen that the $\alpha_1$-AR stimulation exhibits an additional exercise mimetic effect in the cardiac muscle. That is to say, although only the increase in the contractile force of cardiac muscle was known previously through *in-vitro* experiments, an exercise mimetic effect due to AMPK activation and phosphorylation in skeletal and cardiac muscle cells was demonstrated through this example.

**2-2. Effect of $\alpha_1$-AR agonist administration on cardiac function, body weight and blood pressure in animal model *in vivo***

If midodrine administration leads to increase in AMPK, PPAR-δ and PGC-1a in skeletal muscle *in vivo,* it may be thought that the cascade of AMPK-PPAR-δ-PGC-1a activation with proven exercise mimetic effect in the skeletal muscle will also improve the contractility of cardiac muscle. Therefore, cardiac function and body weight were investigated *in vivo.*

**Cardiac function and body weight**

As seen from Table 1 below, the echocardiographic data for 8-week-old rats revealed that the left ventricular performance of the midodrine-administered rat (group II) and the atenolol-administered rat (group III) was higher than that of the unadministered control (group IV).

The left ventricular mass was the lowest for the group II but no significant difference was observed in cardiac mass between the groups. The group III showed the highest body weight.

[Table 1]

| Parameters | Midodrine (group II) | Atenolol (group III) | Control (group IV) | *p*-Value |
|---|---|---|---|---|
| Diastolic left ventricular diaphragm, mm | 1.46 ± 0.13[a] | 1.50 ± 0.13[a] | 1.45± 0.11[a] | 0.3256 |
| Diastolic left ventricular wall, mm | 1.54 ± 0.13[a] | 1.67 ± 0.16 | 1.54 ± 0.15[a] | 0.0046 |
| Diastolic left ventricular inner size, mm | 6.06 ± 0.42[a] | 6.33 ± 0.35[a] | 6.37 ± 0.72[a] | 0.1082 |
| Systolic left ventricular inner size, mm | 3.32 ± 0.54[a] | 3.43 ± 0.28[a] | 3.82 ± 1.01 | 0.0445 |
| Left ventricular fractional shortening, % | 45.48 ± 6.25[a] | 45.70 ± 5.82[a] | 38.77 ± 8.59 | 0.0019 |
| Left ventricular ejection fraction, % | 81.55 ± 6.12[a] | 82.00± 5.31[a] | 73.87 ± 10.13 | 0.0007 |
| Left ventricular mass according to ASE, g | 1.04 ± 0.06 | 1.10 ± 0.07[a] | 1.07 ± 0.12[a] | 0.0313 |
| Body weight, g | 238.24 ± 11.69[a] | 296.46± 16.73[b] | 236.20 ± 7.58[a] | 0.009 |
| Cardiac weight, g | 1.47 ± 0.16[a] | 1.47± 0.15[a] | 1.78 ± 0.35[a] | 0.062 |

**2-3. Expression of AMPK, PPAR-δ and PGC-1α proteins in cardiac muscle, skeletal muscle, aorta and liver**

In order to confirm the presence of $\alpha_1$-AR in skeletal muscle, the expression of the $\alpha_1$-AR protein in skeletal muscle of the 4-week-old basal control group rat (I), the midodrine-administered rat (II), the atenolol-administered rat (III) and the 8-week-old unadministered control group rat (IV) was investigated by western blotting. As shown in FIG. 2a, the group IV showed the highest $\alpha_1$-AR expression.

In addition, the expression of the AMPK, PPAR-δ and PGC-1a proteins in the cardiac muscle, skeletal muscle and liver was investigated by western blotting. As shown in FIG. 2b (cardiac muscle), FIG. 2c (skeletal muscle) and FIG. 2d (liver), in the cardiac muscle, the expression of the phosphorylated AMPK protein was higher for the group I than the group IV ($p < 0.05$). The midodrine-treated group II showed significantly higher AMPK expression in the cardiac muscle and skeletal muscle than the control group IV, whereas the atenolol-treated group III did not show higher AMPK protein expression in the organs as compared to the control group IV of the same age.

It is to be noted that the increase of PGC-1a and PPAR-δ is much larger than the increase of AMPK in the cardiac muscle, whereas the increase of PGC-1a and PPAR-δ is much less than the increase of AMPK in the skeletal muscle. This means that, whereas the exercise mimetic effect induces AMPK activation and PGC-1α and PPAR-δ expression in the cardiac muscle, PGC-1α and PPAR-δ are not increased as much as compared to AMPK in the skeletal muscle.

**2-4. Expression of angiotensin II AT-1 receptor, eNOS, SOD, NADPH, TNFα and MCP genes in aorta and heart**

The mRNA expression level of the genes related with vasoconstriction, nitric oxide production, oxidative stress and inflammation in the aorta and cardiac muscle tissue was investigated by northern blot for the 4-week-old basal rat (I), midodrine-administered rat (II), atenolol-administered rat (III) and 8-week-old unadministered control group rat (IV).

As seen from FIG. 3a, the expression of angiotensin II AT1R mRNA in the aorta was lower for the groups II and III as compared to the two control groups (groups I and IV). The expression of eNOS was the lowest in the group II but no significant difference from other groups was observed. The SOD expression was the highest in the group II. The expression of the NAD(P)H oxidase gp91-phox (Nox-2) mRNA was higher for the group I but there was no difference between the 8-week-old spontaneously hypertensive rats regardless of drug administration. The TNFa expression was the lowest in the group II, moderate in the group III, higher in the group IV and the highest in the group I.

Also, as seen from FIG. 3b, the expression of angiotensin II AT1R mRNA in the cardiac muscle was the lowest in the group II, similarly to the result for the aorta, and moderate in the group III. There was no difference in the eNOS expression among the groups. SOD was expressed at a significantly higher level in the group II as compared to the group IV. In contrast to the aortic (vascular) tissue, there was no difference in the expression of the NAD(P)H oxidase gp91-phox (Nox-2) mRNA between the groups regardless of drug administration. The expression of TNFa in the cardiac muscle was not decreased in response to the midodrine administration.

**2-5. Effect of $\alpha_1$-AR agonist administration on cytokines, reactive oxygen species, adiponectin and fat profile**

The effect of long-term administration of midodrine on the expression of cytokines (IL and TNFa), reactive oxygen species (ROS) and adiponectin and the fat profile was investigated. The result is given in Table 2.

[Table 2]

| | Basal control (Group I) | Midodrine (Group II) | Atenolol (Group III) | Control (Group IV) | p-value |
|---|---|---|---|---|---|
| IL-1β (μg/ml) | 0.22 ± 0.20[a] | 0.46 ± 0.422[a] | 2.02 ± 1.53[a] | 1.23 ± 1.45[a] | 0.194 |
| IL-1α (μg/ml) | 1.94 ± 1.09[a] | 0.14 ± 0.32[b] | 5.51 ± 2.64[c] | 0.15± 0.35[b] | 0.004 |
| IL-6 (μg/ml) | 450 ± 0.20[a] | 4.82 ± 0.49[a] | 5.62 ± 1.42[a] | 5.48 ± 1.15[a] | 0.360 |
| TNP-α (μg/ml) | 3.60 ± 0.65[a] | 2.64 ± 1.64[a] | 8.35 ± 1.36[b] | 5.36±1.94[a] | 0.006 |
| IL-4 (μg/ml) | 1.72 ± 0.26[a] | 1.84 ± 0.21[a] | 2.94 ± 0.90[b] | 2.28 ± 0.50[ab] | 0.030 |
| IL-10 (μg/ml) | 12.34 ± 0.89[a] | 12.15 ± 0.37[a] | 15.33 ± 2.79[b] | 13.52± 1.66[a] | 0.094 |
| ROS (ng/ml) | 186.8 ± 25.7[a] | 204.9 ± 27.5[a] | 223.1 ± 45.4[a] | 254.8 ± 16.8[a] | 0.254 |
| Adiponectin (ng/ml/g) | 74796.6± 13898.0[a] | 7585.8 ± 182.3[b] | 6136.5 ± 574.7[bc] | 5455.8 ±709.7[c] | <0.001 |

As seen from Table 2, the level of the proinflammatory cytokines (IL-1β and IL-6) was not significantly different between the groups. The level of IL-1α was lower in the group II than in the group I but was significantly increased in the group III. The TNF-α expression was significantly increased in the group III as compared to other groups. The expression of IL-4 and IL-10 was slightly higher in group III as compared to other groups.

The level of reactive oxygen species (ROS) was lower in the midodrine-administered group II and the atenolol-administered group III than the 8-week-old unadministered control group IV, although the difference was not statistically significant.

In order to investigate how the $\alpha_1$-AR stimulation activates AMPK, the level of adiponectin was measured. The group II showed a significantly higher serum adiponectin level per the weight of visceral fat as compared to the group IV.

The blood lipid profile was as shown in Table 3. Although total cholesterol, LDL cholesterol and HDL cholesterol were higher in the control groups (groups I and IV) as compared to the drug-administered groups (groups II and IV), there was no significant difference in the triglyceride level between the groups.

[Table 3]

| Lipids, (SI/L) | Basal control (Group I) | Midodrine (Group II) | Atenolol (Group III) | Control (Group IV) | *p*-value |
|---|---|---|---|---|---|
| Total cholesterol | 1.69 ± 0.29 | 0.73 ± 0.15[a] | 1.01 ± 0.27[a] | 1.66 ± 0.17 | <0.001 |
| LDL cholesterol | 0.43 ± 0.06 | 0.15 ± 0.04[a] | 0.15 ± 0.03[a] | 0.40 ± 0.03 | <0.001 |

(continued)

| Lipids, (SI/L) | Basal control (Group I) | Midodrine (Group II) | Atenolol (Group III) | Control (Group IV) | *p*-value |
|---|---|---|---|---|---|
| HDL cholesterol | 0.81 ± 0.08 | 0.49±0.11[a] | 0.52 ± 0.04[a] | 0.87 ± 0.15 | <0.001 |
| Triglyceride | 0.83 ± 0.33[a] | 0.43 ± 0.12[a] | 0.77 ± 0.33[a] | 0.77 ± 0.31[a] | 0.092 |

**2-6. Change in expression of mitochondrial oxidases**

The enzyme activity of succinate dehydrogenase (SDH) which is involved in the mitochondrial oxidation process (TCA cycle) was measured in skeletal muscle. As seen from FIG. 4a, the midodrine-administered group II showed the highest activity.

Also, cytochrome c oxidase which is an enzyme involved in the mitochondrial electron transport chain in the skeletal muscle tissue was immunohistochemically stained. As seen from FIG. 4b, the enzyme was the most increased in the midodrine-administered group II.

From these results, it can be seen that midodrine provides a superior effect in metabolism.

**2-7. Comparison of ATP level in tissues**

The level of ATP in the heart, skeletal muscle and liver was measured by ELISA for the 4-week-old basal rat (I), midodrine-administered rat (II), atenolol-administered rat (III) and 8-week-old unadministered control group rat (IV).

As seen from FIG. 5, the heart tissue of the group treated with midodrine or atenolol showed a higher ATP level than the control group SHRs despite the higher cardiac contraction activity.

**2-8. Diabetes-treating effect**

The effect of midodrine on glucose uptake by insulin in mouse skeletal muscle cells (C2C12 cells) was investigated. As seen from FIG. 6, it was confirmed that the glucose uptake was significantly increased when insulin and midodrine were treated in combination as compared to when insulin was treated alone. Accordingly, it can be seen that midodrine is effective in treating diabetes.

**2-9. Obesity-treating effect**

**Inhibition of adipocyte differentiation**

When preadipocytes (3T3-L1) were treated with midodrine, differentiation into adipocytes was inhibited as shown in FIG. 7a (left bottom). Also, the expression of the PPAR$\delta$, p-AMPK and PGC-1$\alpha$ proteins, which inhibit fat synthesis and accumulation, was increased as shown in FIG. 7b.

**Reduction of body weight and abdominal fat**

Body weight and abdominal fat were measured and compared for the midodrine-administered rat (II), atenolol-administered rat (III) and 8-week-old unadministered hypertension control group rat (IV). As seen from FIG. 8, the midodrine administration resulted in reduction of body weight and abdominal fat.

Accordingly, it can be seen from these results that midodrine is effective in treating obesity.

**2-10. Inflammation-treating effect**

When the Raw 264.7 macrophages were treated with midodrine, as seen from FIG. 9, the expression of PPAR$\delta$, AMPK$\alpha_1$ and mannose receptor (MR) mRNAs was increased (A) and the expression of the hexokinase II protein was decreased (B). Also, the increased expression of mannose receptor in macrophages localized in the subcapsular portion of the spleen was confirmed.

Because the expression of the mannose receptor (MR), which is the M2 phenotype receptor exhibiting anti-inflammatory effect in inflammation-related macrophages, was increased whereas the expression of hexokinase II, which is increased in M1 phenotype macrophages, was decreased, it can be seen that midodrine is effective in treating inflammations.

**Example 3: Effect of various $\alpha_1$-adrenergic receptor agonists**

Compounds similar to midodrine were newly synthesized as $\alpha_1$-AR agonists and their effect of inducing AMPK activation and PPAR-$\delta$ and PGC-1a expression in skeletal muscle cells by stimulating $\alpha_1$-AR was confirmed. It was verified that the various $\alpha_1$-AR agonists can induce an exercise mimetic effect.

Compound 5: 2-amino-N-(2-(2,5-dimethoxyphenyl)-2-hydroxyethyl)-3-phenylpropanamide
Compound 7: 2-amino-N-(2-(2,5-dimethoxyphenyl)-2-hydroxyethyl)propanamide
Compound 8: 2-amino-N-(2-(3,5-dimethoxyphenyl)-2-hydroxyethyl)acetamide hydrochloride
Compound 9: 2-amino-N-(2-(5-ethyl-2-methoxyphenyl)-2-hydroxyethyl)acetamide hydrochloride
Compound 10: 2-amino-N-(2-hydroxy-2-phenylethyl)acetamide hydrochloride

Specifically, after treating differentiating C2C12 cells with the compounds at different concentrations (10 $\mu$M, 30 $\mu$M, 50 $\mu$M), the expression of activated (phosphorylated) AMPK (p-AMPK), PPAR-$\delta$ and PGC-1$\alpha$ was investigated by western blot.
As seen from FIG. 10, Compound 5 showed significantly increased expression of activated (phosphorylated) AMPK (p-AMPK) and PPAR-$\delta$ at the intermediate concentration of 30 $\mu$M as compared to the control group (untreated group).
As seen from FIG. 11, Compound 7 showed concentration-dependent increase in the expression of p-AMPK and PPAR-$\delta$ proteins. In particular, for p-AMPK, the 10 $\mu$M treatment group and the 50 $\mu$M treatment group showed significant increase as compared to the control group. For PPAR-$\delta$, the 30 $\mu$M treatment group showed significant increase as compared to the control group.
As seen from FIG. 12, Compound 8 showed overall increase of p-AMPK, PPAR-$\delta$ and PGC-1$\alpha$. In particular, the expression of p-AMPK and PGC-1$\alpha$ was significantly increased at the concentration of 50 $\mu$M.
As seen from FIG. 13, Compound 9 showed overall increase of p-AMPK, PPAR-$\delta$ and PGC-1$\alpha$. In particular, the expression of p-AMPK and PGC-1$\alpha$ was significantly increased at the concentrations of 30 $\mu$M and 50 $\mu$M.
As seen from FIG. 14, Compound 10 showed significantly increased p-AMPK expression at 30 $\mu$M and 50 $\mu$M and significantly increased PPAR$\delta$ expression at 10 $\mu$M and 50 $\mu$M.
According to the above results, it can be seen that $\alpha_1$-AR stimulation by the $\alpha_1$-AR agonist induces an exercise mimetic effect in skeletal muscle, liver and blood vessels even without exercise, improves left ventricular ejection fraction without cardiac hypertrophy or blood pressure increase, reduces inflammation and changes biochemical responses during the initial hypertensive development of spontaneously hypertensive rat. This is directly related with stimulated cardiac muscle contraction, cardiac motion-like effect and AMPK activation.

## Claims

1. A pharmaceutical composition for use in the treatment of a disease requiring the activation of an AMP-activated protein kinase (AMPK), comprising an $\alpha_1$-adrenergic receptor agonist or a pharmaceutically acceptable salt thereof as an active ingredient selected from the group consisting of midodrine, 2-amino-N-(2-(2,5-dimethoxyphenyl)-2-hydroxyethyl)-3-phenylpropanamide, 2-amino-N-(2-(2,5-dimethoxyphenyl)-2-hydroxyethyl)propanamide, 2-amino-N-(2-(3,5-dimethoxyphenyl)-2-hydroxyethyl)acetamide hydrochloride, 2-amino-N-(2-(5-ethyl-2-methoxyphenyl)-2-hydroxyethyl)acetamide hydrochloride and 2-amino-N-(2-hydroxy-2-phenylethyl)acetamide hydrochloride,
wherein the disease is selected from hypertension hyperlipidemia, diabetes, obesity, arteriosclerosis, fatty liver and inflammatory disease.

2. The pharmaceutical composition for the use according to claim 1, wherein the $\alpha_1$-adrenergic receptor agonist induces the activation of AMPK.

3. The pharmaceutical composition for the use according to claim 1, wherein the $\alpha_1$-adrenergic receptor agonist induces the expression of PPAR-$\delta$ or PGC-1$\alpha$.

4. A method for screening a drug for inducing an exercise mimetic effect comprising:

(a) a step of treating a cell with an $\alpha_1$-adrenergic receptor agonist *in vitro;* and
(b) a step of measuring the expression of phosphorylated AMPK (AMP-activated protein kinase), PPAR-$\delta$ (peroxisome proliferator-activated receptor-$\delta$) or PGC-1$\alpha$ (peroxisome proliferator-activated receptor gamma coactivator-1$\alpha$).

5. The screening method according to claim 4, which further comprises (c) a step of selecting the agonist as a drug when the expression of phosphorylated AMPK, PPAR-δ or PGC-1α is increased as compared to a group not treated with the agonist.

6. The screening method according to claim 4, wherein, in the step (b), the expression is measured by western blotting, antigen immunoprecipitation, ELISA, mass spectrometry, RT-PCR, competitive RT-PCR, real-time RT-PCR, RPA (RNase protection assay) or northern blotting.

7. The screening method according to claim 4, wherein, in the step (a), the cell is the cell of a tissue selected from a group comprising skeletal muscle, cardiac muscle, liver, fat and pancreas.

8. The screening method according to claim 4, wherein the exercise mimetic effect is an effect of treating a disease selected from a group comprising a metabolic disease, a cardiovascular disease and an inflammatory disease, and wherein the metabolic disease is a disease selected from a group comprising hyperlipidemia, diabetes, obesity, arteriosclerosis and fatty liver.

**Patentansprüche**

1. Pharmazeutische Zusammensetzung zur Verwendung bei der Behandlung einer Krankheit, welche die Aktivierung einer AMP-aktivierten Proteinkinase (AMPK) erfordert, umfassend einen $\alpha_1$-adrenergen Rezeptoragonisten oder ein pharmazeutisch verträgliches Salz davon als Wirkstoff, ausgewählt aus der Gruppe bestehend aus Midodrin, 2-Amino-N-(2-(2,5-dimethoxyphenyl)-2-hydroxyethyl)-3-phenylpropanamid, 2-Amino-N-(2-(2,5-dimethoxyphenyl)-2-hydroxyethyl)propanamid, 2-Amino-N-(2-(3,5-dimethoxyphenyl)-2-hydroxyethyl)acetamidhydrochlorid, 2-Amino-N-(2-(5-ethyl-2-methoxyphenyl)-2-hydroxyethyl)acetamidhydrochlorid und 2-Amino-N-(2-hydroxy-2-phenylethyl)-acetamidhydrochlorid,
wobei die Krankheit aus Bluthochdruck, Hyperlipidämie, Diabetes, Fettleibigkeit, Arteriosklerose, Fettleber und entzündlicher Erkrankung ausgewählt ist.

2. Pharmazeutische Zusammensetzung zur Verwendung gemäß Anspruch 1, wobei der $\alpha_1$-adrenerge Rezeptoragonist die Aktivierung von AMPK induziert.

3. Pharmazeutische Zusammensetzung zur Verwendung gemäß Anspruch 1, wobei der $\alpha_1$-adrenerge Rezeptoragonist die Expression von PPAR-δ oder PGC-1α induziert.

4. Verfahren zum Screenen eines Arzneimittels zum Induzieren einer übungsmimetischen Wirkung, umfassend:

(a) einen Schritt der Behandlung einer Zelle mit einem $\alpha_1$-adrenergen Rezeptoragonisten *in vitro;* und
(b) einen Schritt des Messens der Expression von phosphorylierter AMPK (AMPaktivierte Proteinkinase), PPAR-δ (Peroxisom-Proliferator-aktivierter Rezeptor-δ) oder PGC-1a (Peroxisom-Proliferator-aktivierter Rezeptor-Gamma-Coaktivator-1α).

5. Screening-Verfahren gemäß Anspruch 4, das ferner (c) einen Schritt des Auswählens des Agonisten als Arzneimittel umfasst, wenn die Expression von phosphorylierter AMPK, PPAR-δ oder PGC-1α im Vergleich zu einer nicht mit dem Agonisten behandelten Gruppe erhöht ist.

6. Screening-Verfahren gemäß Anspruch 4, wobei im Schritt (b) die Expression durch Western Blotting, Antigen-Immunpräzipitation, ELISA, Massenspektrometrie, RT-PCR, kompetitive RT-PCR, Echtzeit-RT-PCR, RPA (RNase-Schutz-Assay) oder Northern Blotting gemessen wird.

7. Screening-Verfahren gemäß Anspruch 4, wobei im Schritt (a) die Zelle die Zelle eines Gewebes ist, das aus einer Gruppe, umfassend Skelettmuskulatur, Herzmuskulatur, Leber, Fett und Pankreas, ausgewählt ist.

8. Screening-Verfahren gemäß Anspruch 4, wobei die übungsmimetische Wirkung eine Wirkung der Behandlung einer Krankheit ist, die aus einer Gruppe, umfassend eine Stoffwechselerkrankung, eine Herz-Kreislauf-Erkrankung und eine entzündliche Erkrankung, ausgewählt ist, und wobei die Stoffwechselerkrankung eine Krankheit ist, die aus einer Gruppe, umfassend Hyperlipidämie, Diabetes, Fettleibigkeit, Arteriosklerose und Fettleber, ausgewählt ist.

## Revendications

**1.** Composition pharmaceutique destinée à être utilisée dans le traitement d'une maladie nécessitant l'activation d'une protéine kinase activée par AMP (AMPK), comprenant un agoniste de récepteur $\alpha_1$ adrénergique ou un sel pharmaceutiquement acceptable de celui-ci en tant qu'ingrédient actif choisi dans le groupe comprenant midodrine, 2-amino-N-(2-(2,5-diméthoxyphényl)-2-hydroxyéthyl)-3-phénylepropanamide, 2-amino-N-(2-(2,5-diméthoxyphényl)-2-hydroxyéthyl)propanamide, chlorhydrate de 2-amino-N-(2-(3,5-diméthoxyphényl)-2-hydroxyéthyl)acétamide, chlorhydrate de 2-amino-N-(2-(5-éthyl-2-méthoxyphényl)-2-hydroxyéthyl)acétamide et chlorhydrate de 2-amino-N-(2-hydroxy-2-phényléthyle)acétamide,
dans laquelle la maladie est choisie parmi l'hypertension et l'hyperlipidémie, le diabète, l'obésité, l'artériosclérose, la stéatose hépatique et une maladie inflammatoire.

**2.** Composition pharmaceutique à utiliser selon la revendication 1, dans laquelle l'agoniste de récepteur $\alpha_1$ adrénergique induit l'activation d'AMPK.

**3.** Composition pharmaceutique à utiliser selon la revendication 1, dans laquelle l'agoniste de récepteur $\alpha_1$ adrénergique induit l'expression de PPAR-$\delta$ ou de PGC-1$\alpha$.

**4.** Procédé de criblage d'un médicament pour induire un effet de mimétisme d'exercice, comprenant :

(a) une étape de traitement d'une cellule avec un agoniste de récepteur $\alpha_1$ adrénergique *in vitro* ; et
(b) une étape de mesure de l'expression d'AMPK phosphorylée (protéine kinase activée par AMP), de PPAR-$\delta$ (récepteur-$\delta$ activé par proliférateur de peroxysome) ou de PGC-1$\alpha$ (coactivateur 1$\alpha$ gamma de récepteur activé par proliférateur de peroxysome).

**5.** Procédé de criblage selon la revendication 4, qui comprend en outre (c) une étape de sélection de l'agoniste en tant que médicament lorsque l'expression d'AMPK phosphorylée, de PPAR-$\delta$ ou de PGC-1$\alpha$ est augmentée par rapport à un groupe non traité avec l'agoniste.

**6.** Procédé de criblage selon la revendication 4, dans lequel, dans l'étape (b), l'expression est mesurée par buvardage de western, immunoprécipitation d'antigène, ELISA, spectrométrie de masse, RT-PCR, RT-PCR compétitive, RT-PCR en temps réel, RPA (test de protection RNase) ou buvardage de northern.

**7.** Procédé de criblage selon la revendication 4, dans lequel, dans l'étape (a), la cellule est la cellule d'un tissu choisi dans un groupe comprenant muscle squelettique, muscle cardiaque, foie, graisse et pancréas.

**8.** Procédé de criblage selon la revendication 4, dans lequel l'effet de mimétisme d'exercice est un effet de traitement d'une maladie choisie dans un groupe comprenant une maladie métabolique, une maladie cardiovasculaire et une maladie inflammatoire, et dans lequel la maladie métabolique est une maladie choisie dans un groupe comprenant l'hyperlipidémie, le diabète, l'obésité, l'artériosclérose et la stéatose hépatique.

FIG. 1

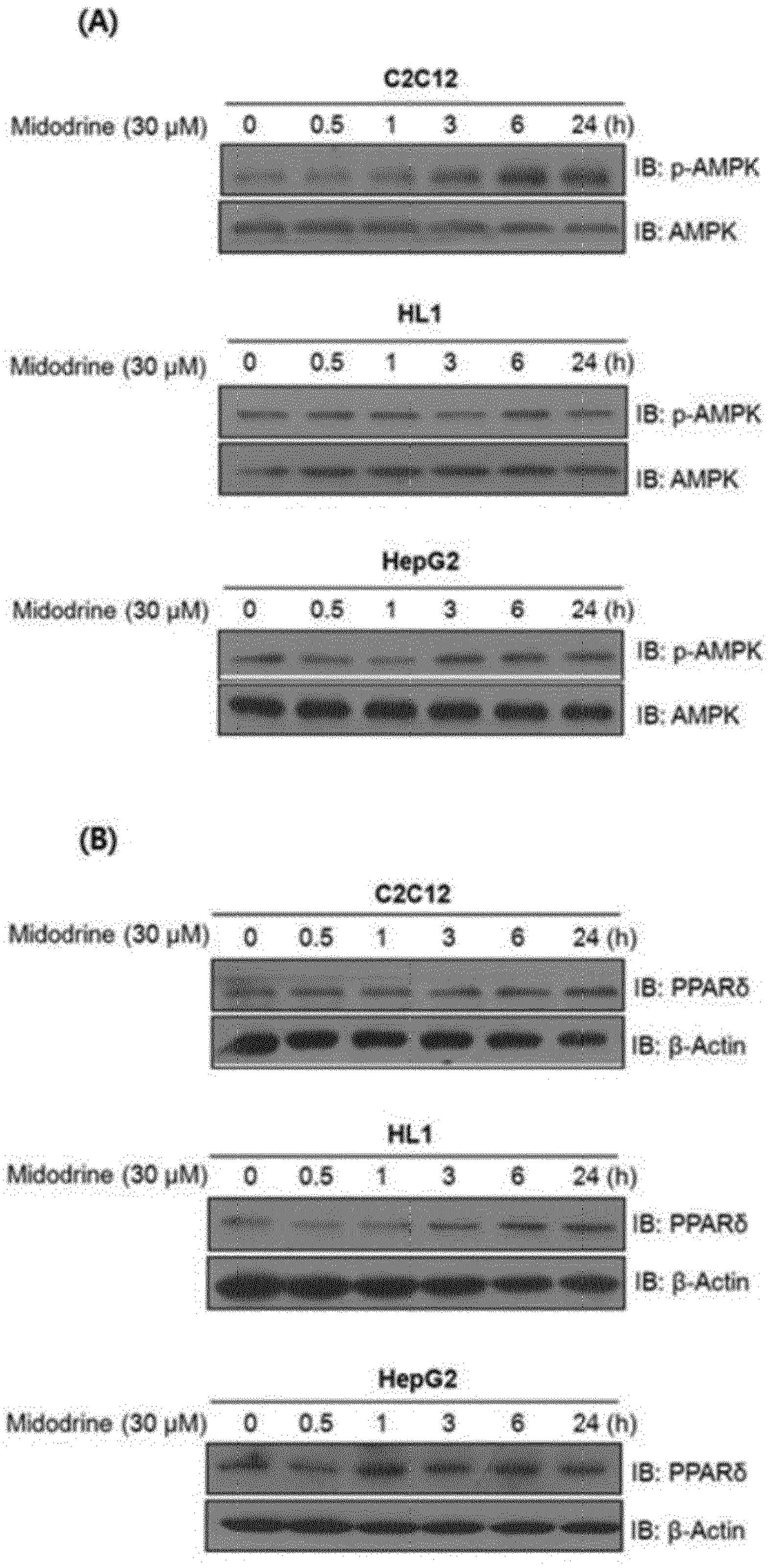
(A)
C2C12
Midodrine (30 μM) 0 0.5 1 3 6 24 (h)
IB: p-AMPK
IB: AMPK
HL1
Midodrine (30 μM) 0 0.5 1 3 6 24 (h)
IB: p-AMPK
IB: AMPK
HepG2
Midodrine (30 μM) 0 0.5 1 3 6 24 (h)
IB: p-AMPK
IB: AMPK
(B)
C2C12
Midodrine (30 μM) 0 0.5 1 3 6 24 (h)
IB: PPARδ
IB: β-Actin
HL1
Midodrine (30 μM) 0 0.5 1 3 6 24 (h)
IB: PPARδ
IB: β-Actin
HepG2
Midodrine (30 μM) 0 0.5 1 3 6 24 (h)
IB: PPARδ
IB: β-Actin

FIG. 2a

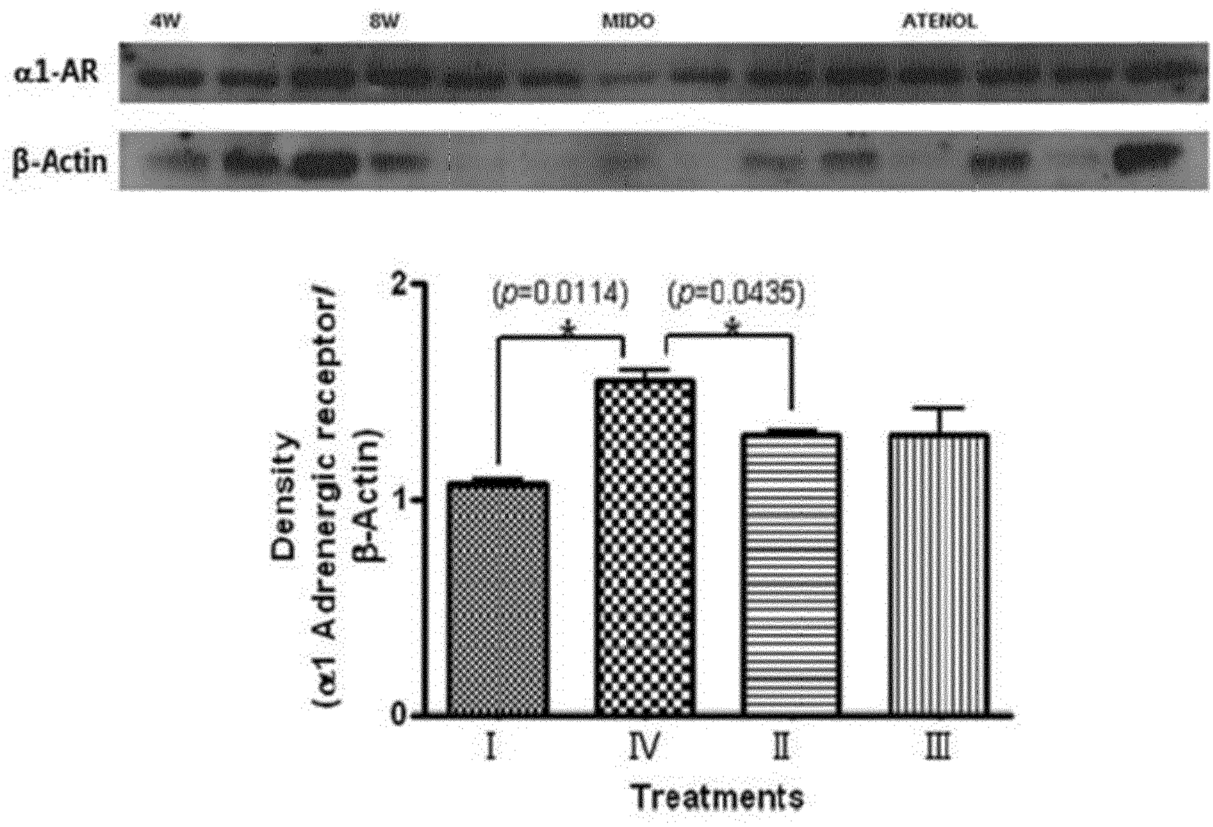
4W
8W
MIDO
ATENOL
α1-AR
β-Actin
Density
(α1 Adrenergic receptor/
β-Actin)
(p=0.0114)
(p=0.0435)
*
*
2
1
0
I
IV
II
III
Treatments

FIG. 2b

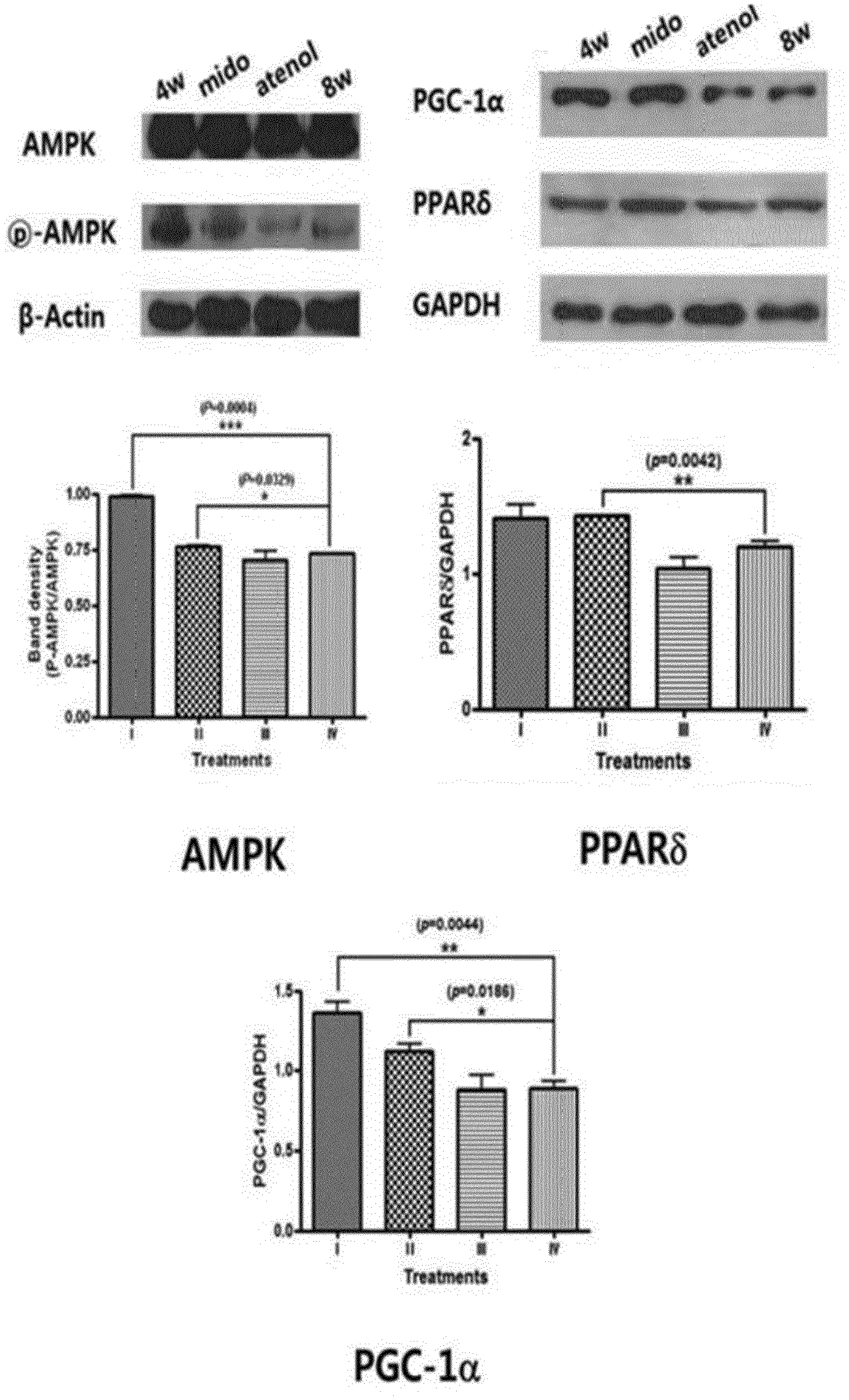
4w
mido
atenol
8w
AMPK
p-AMPK
β-Actin
PGC-1α
PPARδ
GAPDH
Band density (P-AMPK/AMPK)
Treatments
AMPK
PPARδ/GAPDH
(p=0.0042)
**
Treatments
PPARδ
(p=0.0044)
**
(p=0.0186)
*
PGC-1α/GAPDH
Treatments
PGC-1α

FIG. 2c

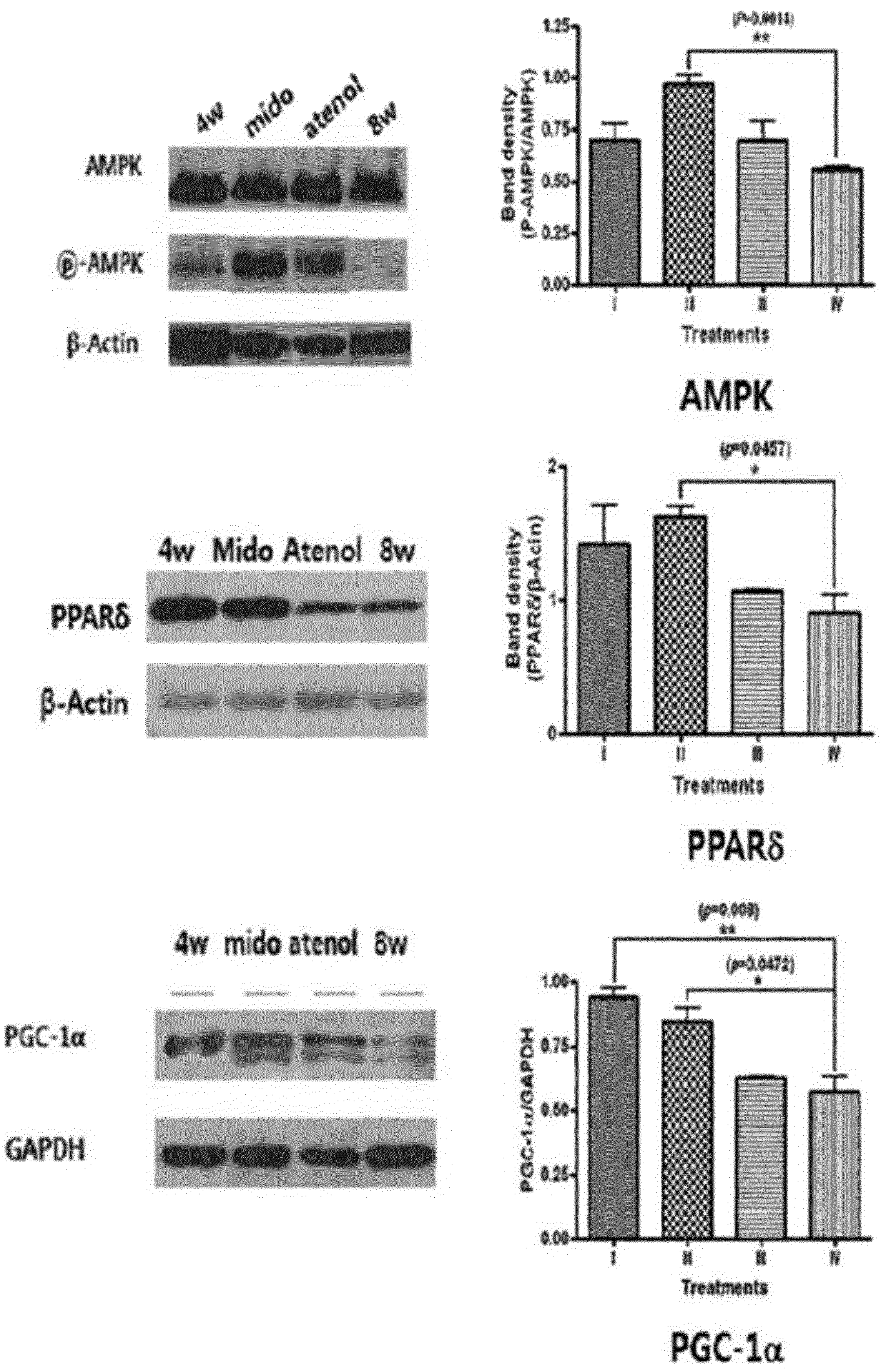
4w
mido
atenol
8w
AMPK
ⓟ-AMPK
β-Actin
Band density (P-AMPK/AMPK)
Treatments
AMPK
4w Mido Atenol 8w
PPARδ
β-Actin
(p=0.0457)
Band density (PPARδ/β-Acin)
Treatments
PPARδ
4w mido atenol 8w
PGC-1α
GAPDH
(p=0.008)
(p=0.0472)
PGC-1α/GAPDH
Treatments
PGC-1α

FIG. 2d

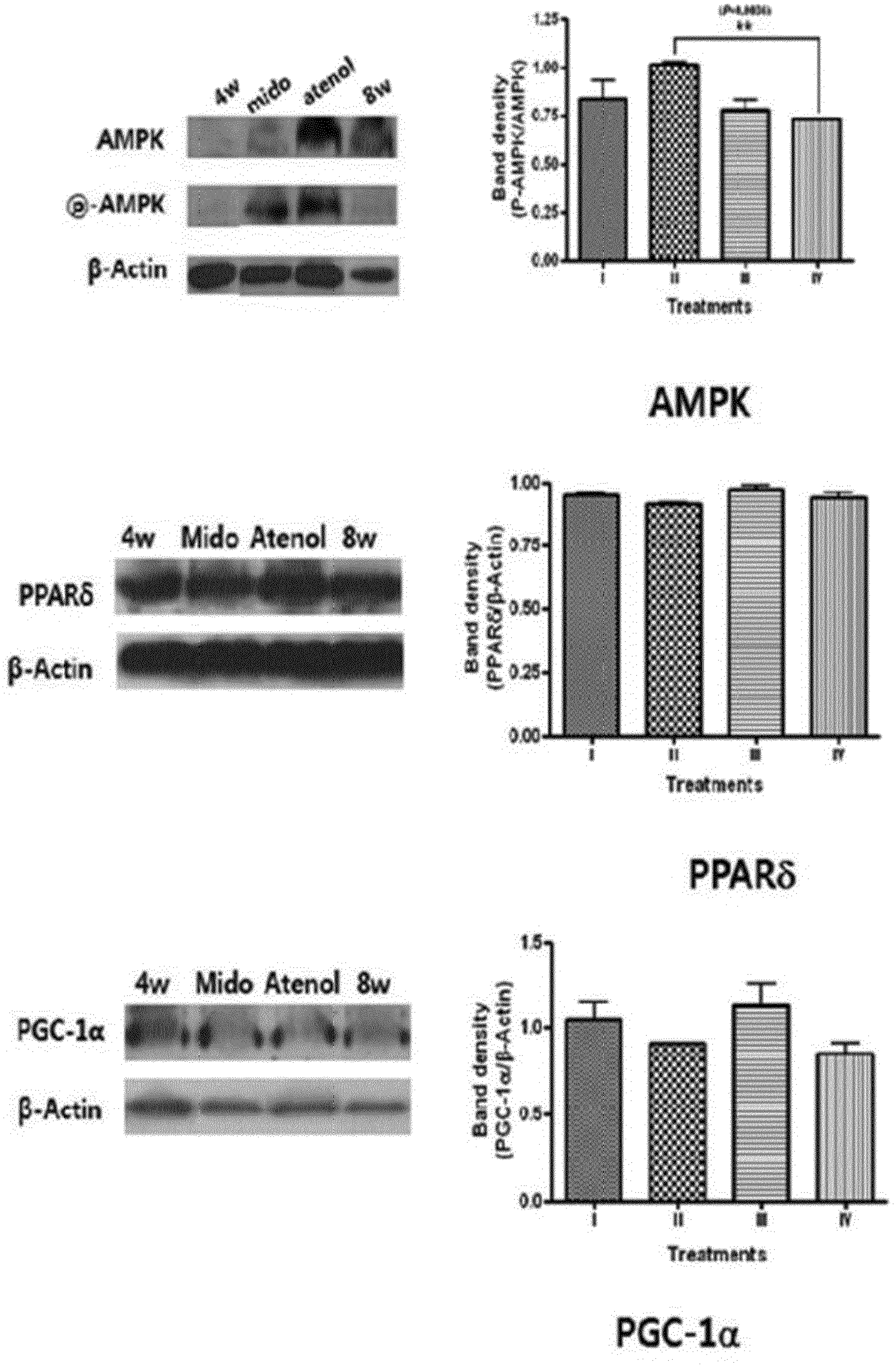
4w mido atenol 8w
AMPK
℗-AMPK
β-Actin
Band density (P-AMPK/AMPK)
1.25
1.00
0.75
0.50
0.25
0.00
I II III IV
Treatments
AMPK
4w Mido Atenol 8w
PPARδ
β-Actin
Band density (PPARδ/β-Actin)
1.00
0.75
0.50
0.25
0.00
I II III IV
Treatments
PPARδ
4w Mido Atenol 8w
PGC-1α
β-Actin
Band density (PGC-1α/β-Actin)
1.5
1.0
0.5
0.0
I II III IV
Treatments
PGC-1α

FIG. 3a

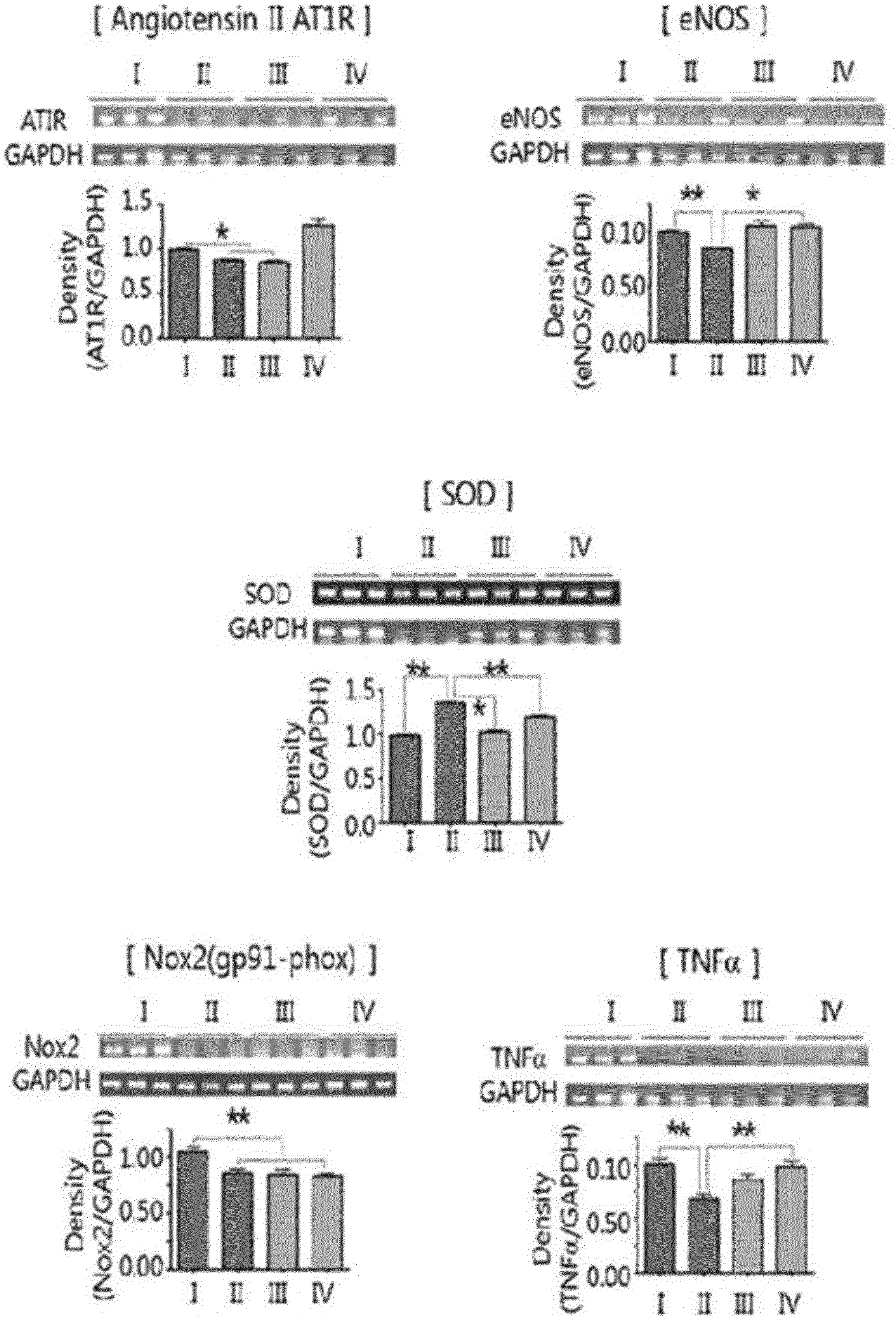
[ Angiotensin II AT1R ]
I II III IV
ATIR
GAPDH
Density (AT1R/GAPDH)
1.5
1.0
0.5
0.0
*
I II III IV
[ eNOS ]
I II III IV
eNOS
GAPDH
Density (eNOS/GAPDH)
0.10
0.50
0.00
**
*
I II III IV
[ SOD ]
I II III IV
SOD
GAPDH
Density (SOD/GAPDH)
1.5
1.0
0.5
0.0
**
**
*
I II III IV
[ Nox2(gp91-phox) ]
I II III IV
Nox2
GAPDH
Density (Nox2/GAPDH)
1.00
0.50
0.00
**
I II III IV
[ TNFα ]
I II III IV
TNFα
GAPDH
Density (TNFα/GAPDH)
0.10
0.50
0.00
**
**
I II III IV

FIG. 3b

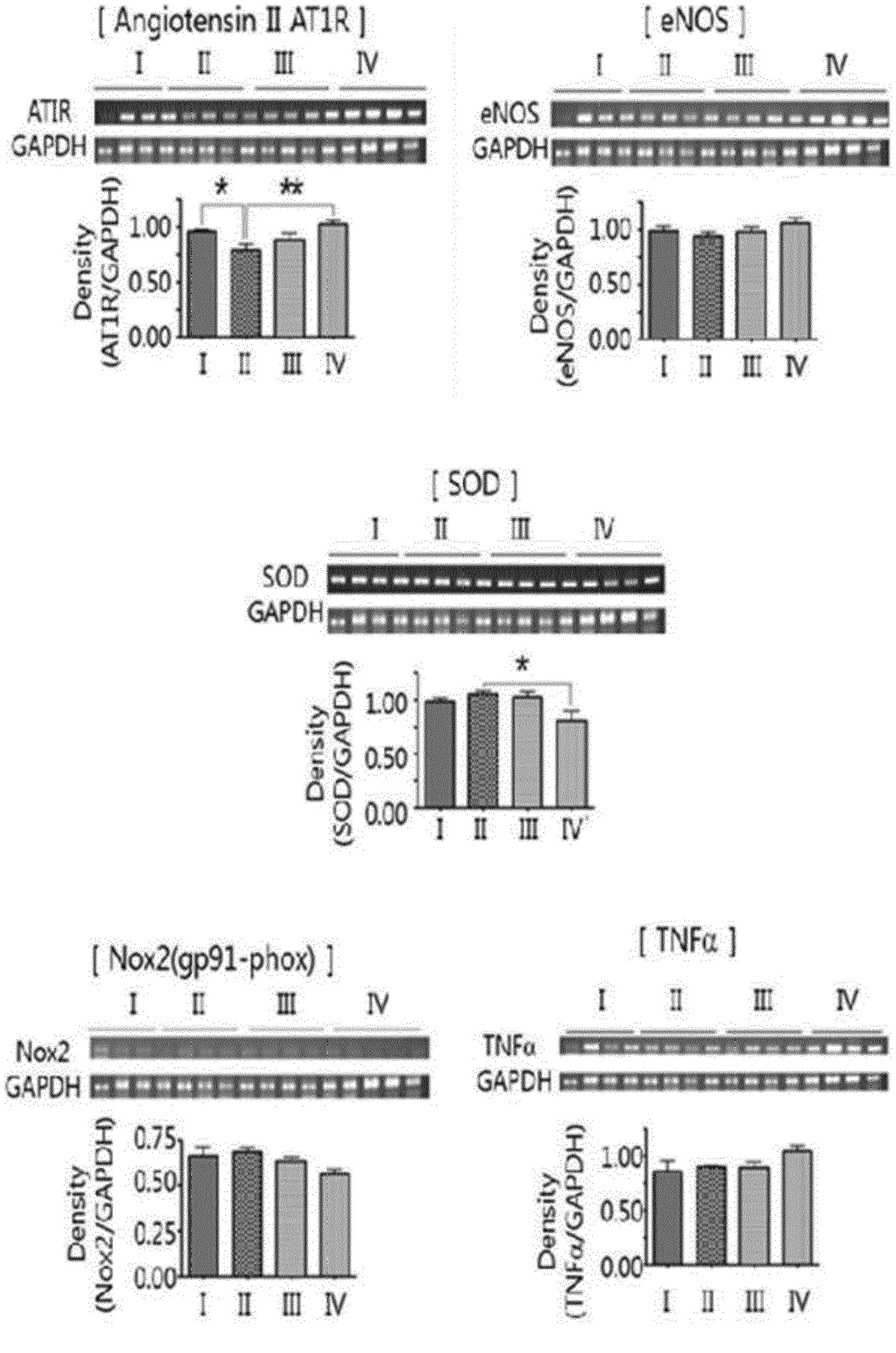
[ Angiotensin II AT1R ]
I II III IV
ATIR
GAPDH
Density (AT1R/GAPDH)
1.00
0.50
0.00
*
**
I II III IV
[ eNOS ]
I II III IV
eNOS
GAPDH
Density (eNOS/GAPDH)
1.00
0.50
0.00
I II III IV
[ SOD ]
I II III IV
SOD
GAPDH
Density (SOD/GAPDH)
1.00
0.50
0.00
*
I II III IV
[ Nox2(gp91-phox) ]
I II III IV
Nox2
GAPDH
Density (Nox2/GAPDH)
0.75
0.50
0.25
0.00
I II III IV
[ TNFα ]
I II III IV
TNFα
GAPDH
Density (TNFα/GAPDH)
1.00
0.50
0.00
I II III IV

FIG. 4a

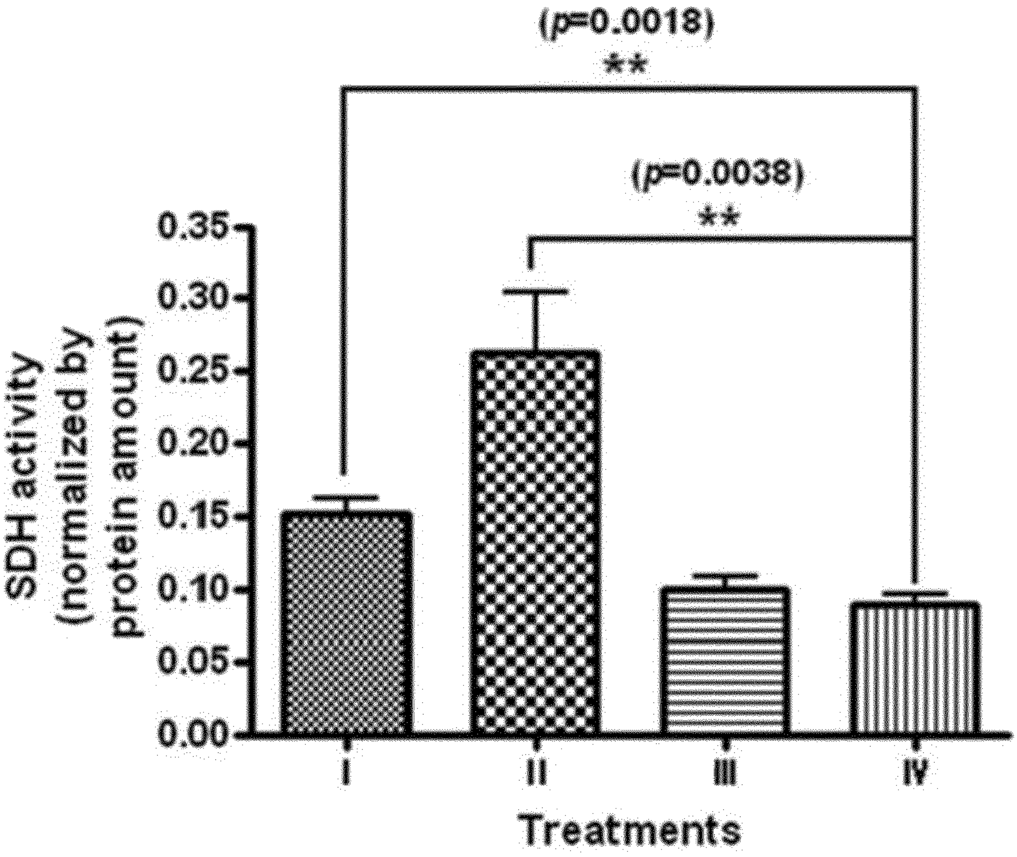
(p=0.0018)
**
(p=0.0038)
**
0.35
0.30
0.25
0.20
0.15
0.10
0.05
0.00
SDH activity (normalized by protein amount)
I
II
III
IV
Treatments

FIG. 4b

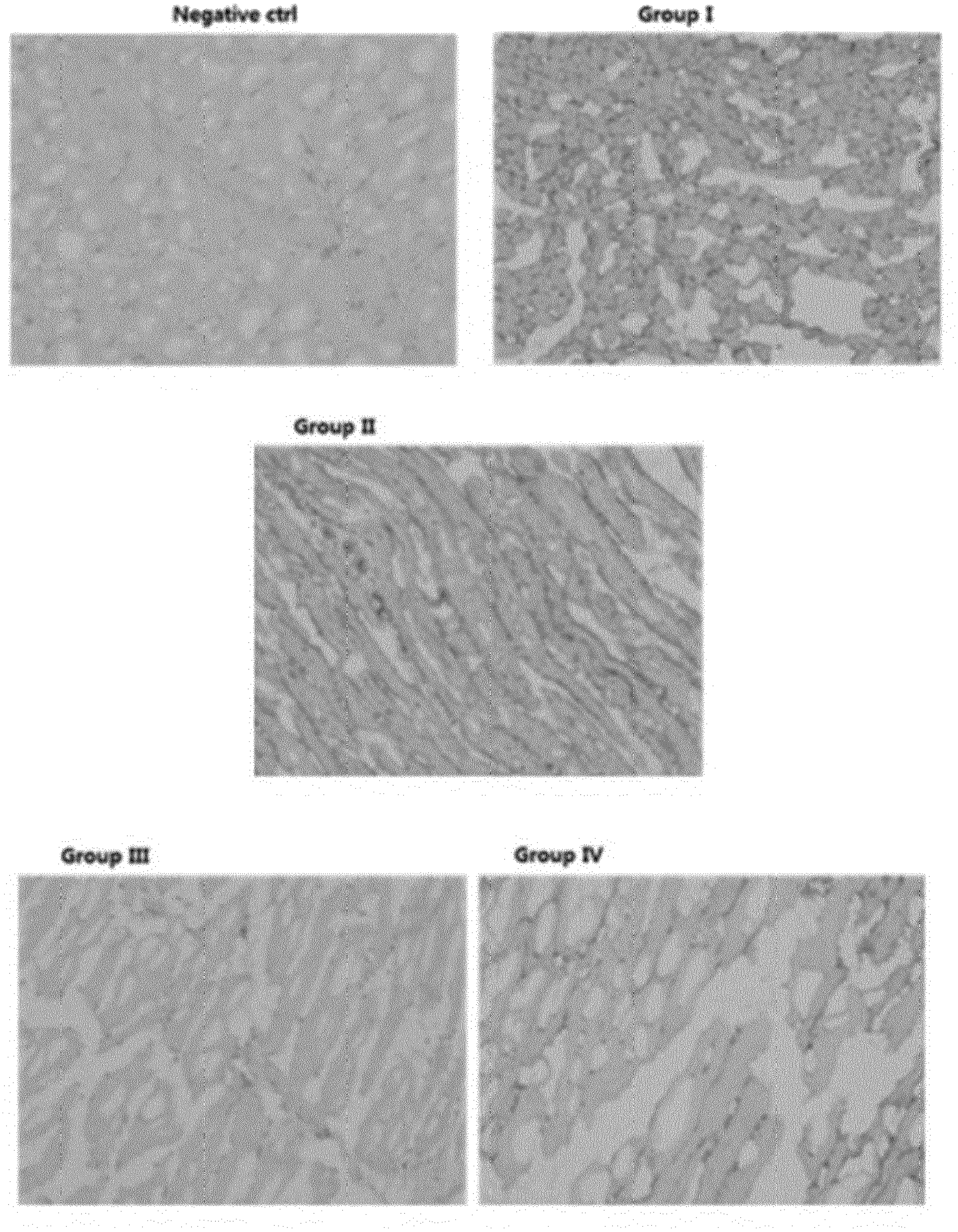

FIG. 5

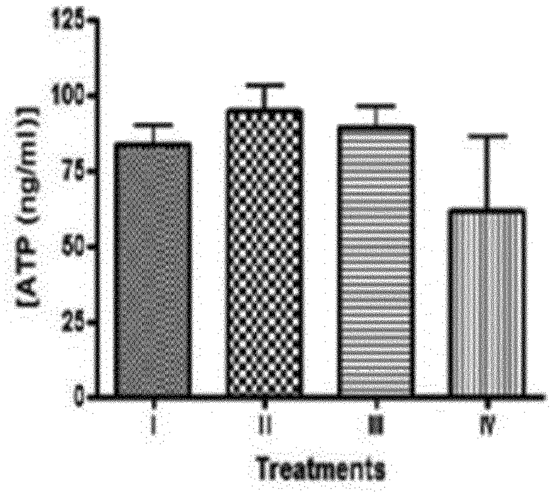


Heart

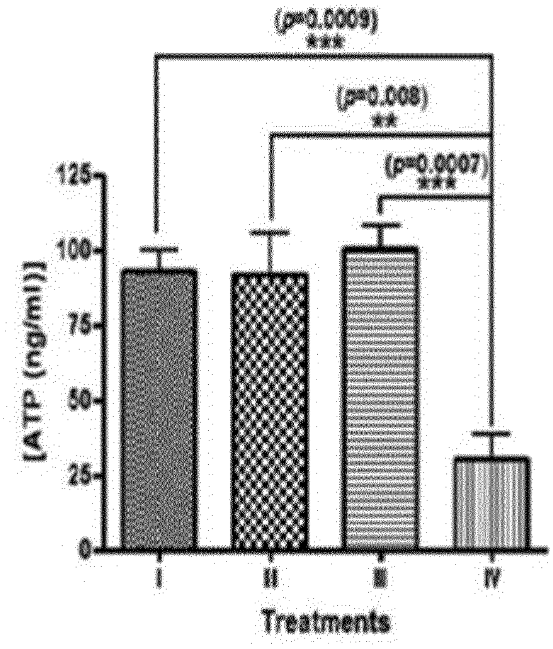


Skeletal Muscle

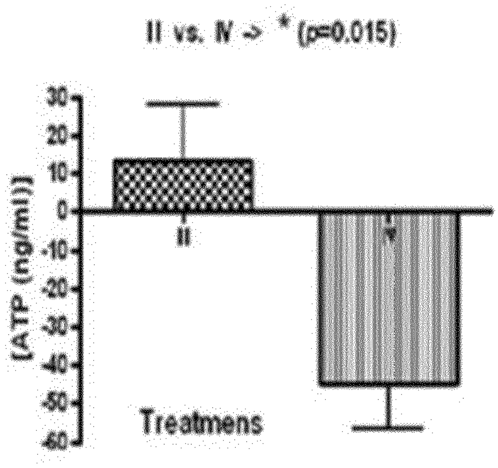


Liver

FIG. 6

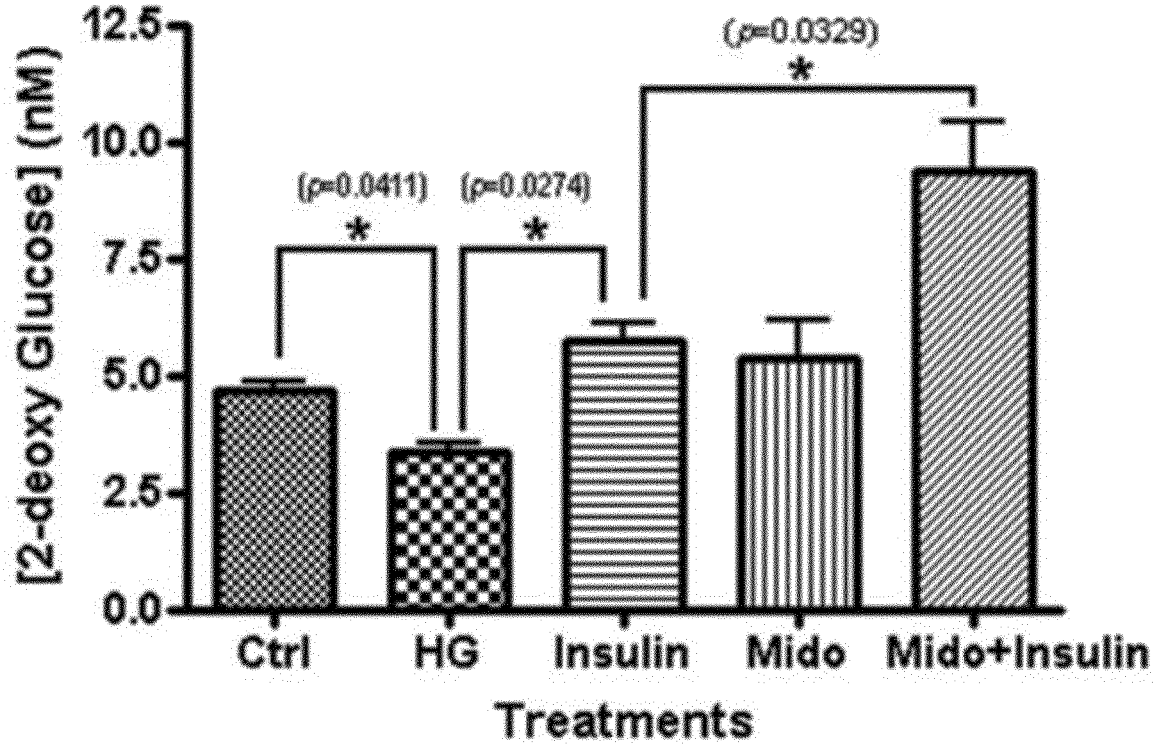

[2-deoxy Glucose] (nM)
12.5
10.0
7.5
5.0
2.5
0.0
(p=0.0411)
(p=0.0274)
(p=0.0329)
*
*
*
Ctrl
HG
Insulin
Mido
Mido+Insulin
Treatments

FIG. 7a

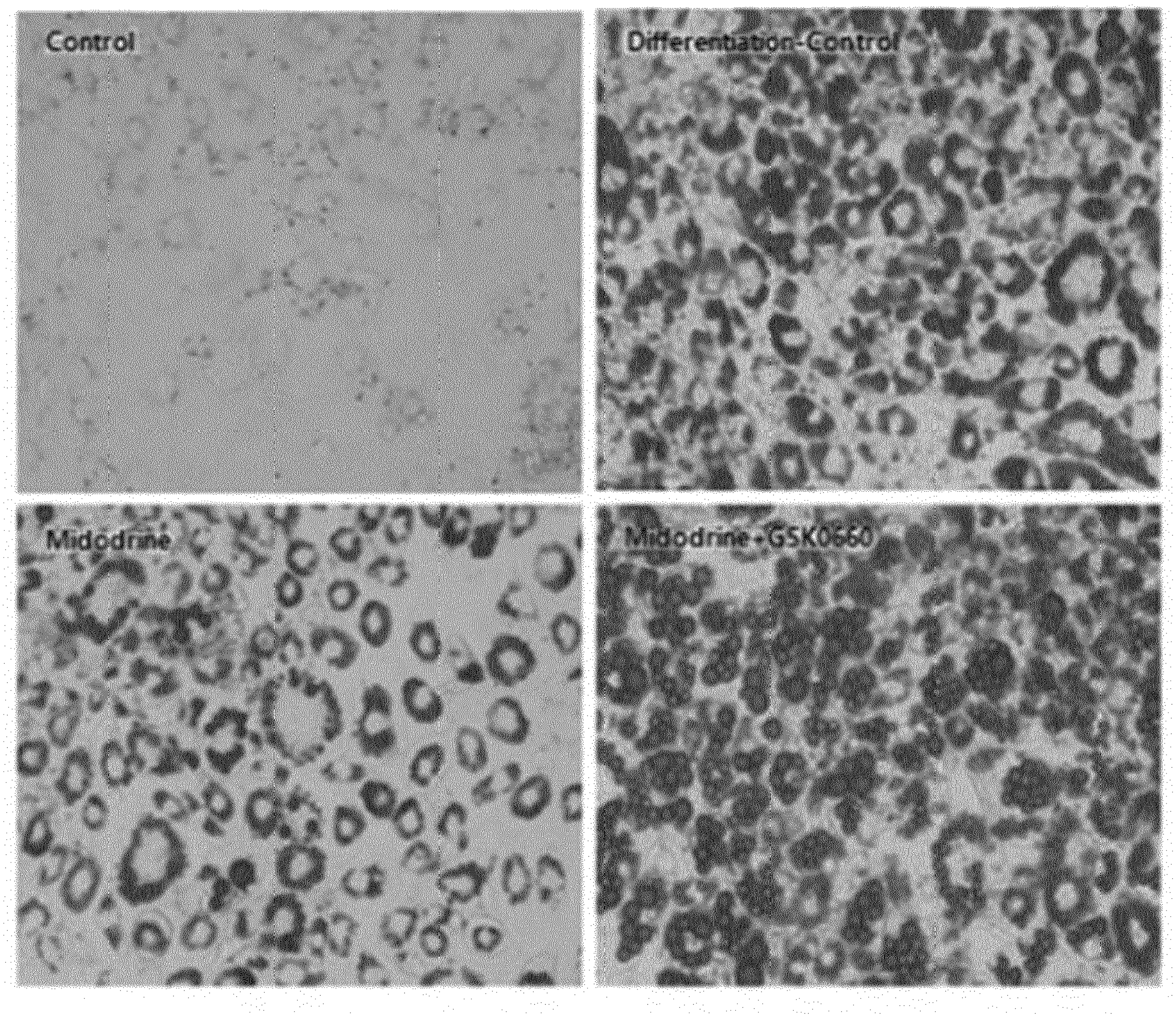
Control
Differentiation-Control
Midodrine
Midodrine+GSK0660

FIG. 7b

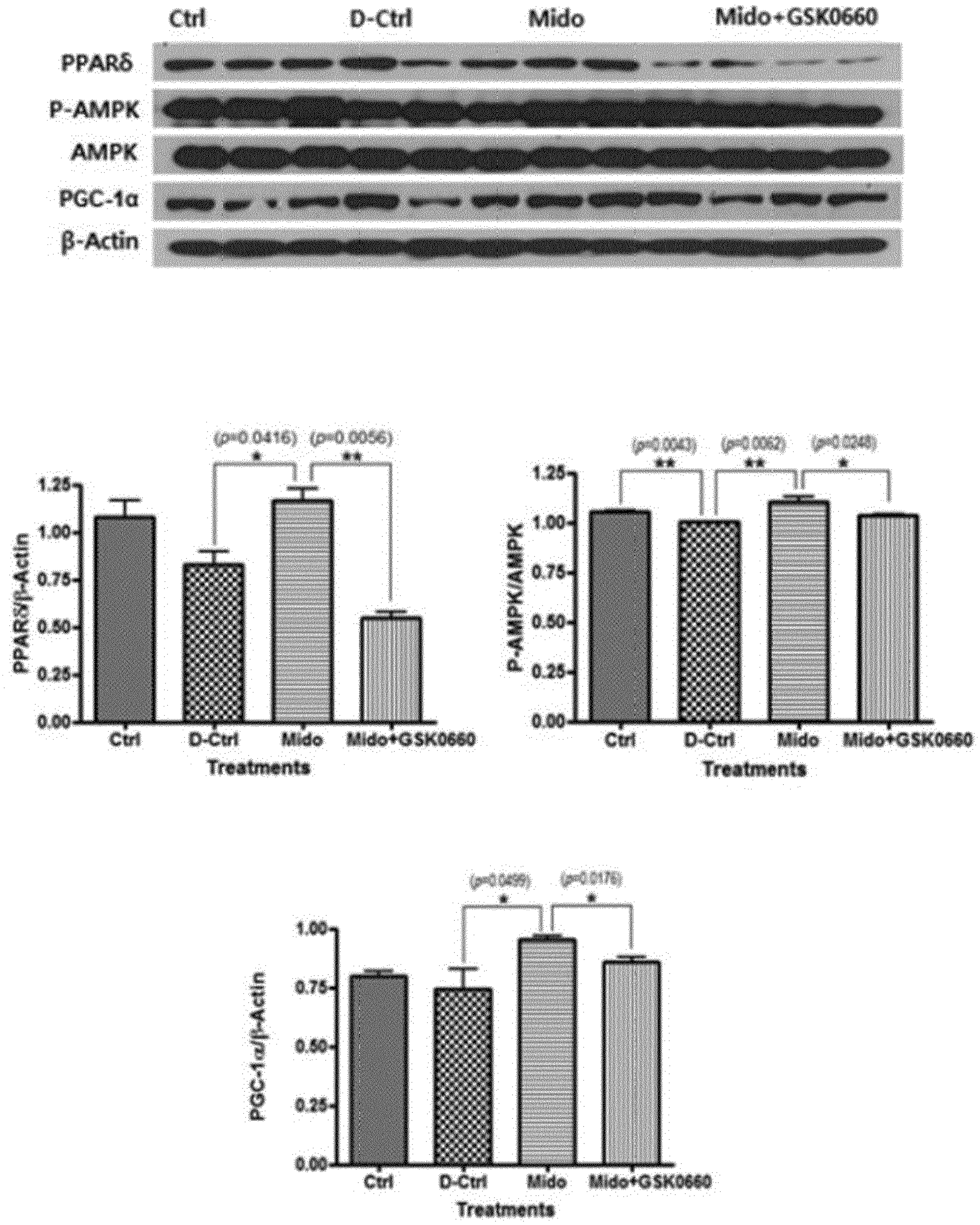
Ctrl
D-Ctrl
Mido
Mido+GSK0660
PPARδ
P-AMPK
AMPK
PGC-1α
β-Actin
(p=0.0416)
(p=0.0056)
*
**
PPARδ/β-Actin
1.25
1.00
0.75
0.50
0.25
0.00
Ctrl
D-Ctrl
Mido
Mido+GSK0660
Treatments
(p=0.0043)
(p=0.0062)
(p=0.0248)
**
**
*
P-AMPK/AMPK
1.25
1.00
0.75
0.50
0.25
0.00
Ctrl
D-Ctrl
Mido
Mido+GSK0660
Treatments
(p=0.0499)
(p=0.0176)
*
*
PGC-1α/β-Actin
1.00
0.75
0.50
0.25
0.00
Ctrl
D-Ctrl
Mido
Mido+GSK0660
Treatments

FIG. 8

(A)

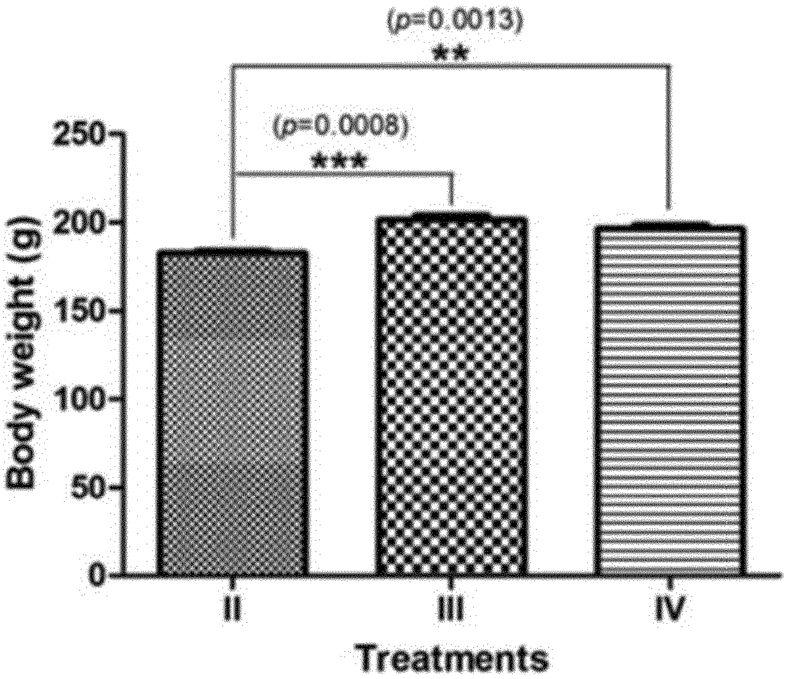
(p=0.0013)
**
(p=0.0008)
***
Body weight (g)
250
200
150
100
50
0
II
III
IV
Treatments

(B)

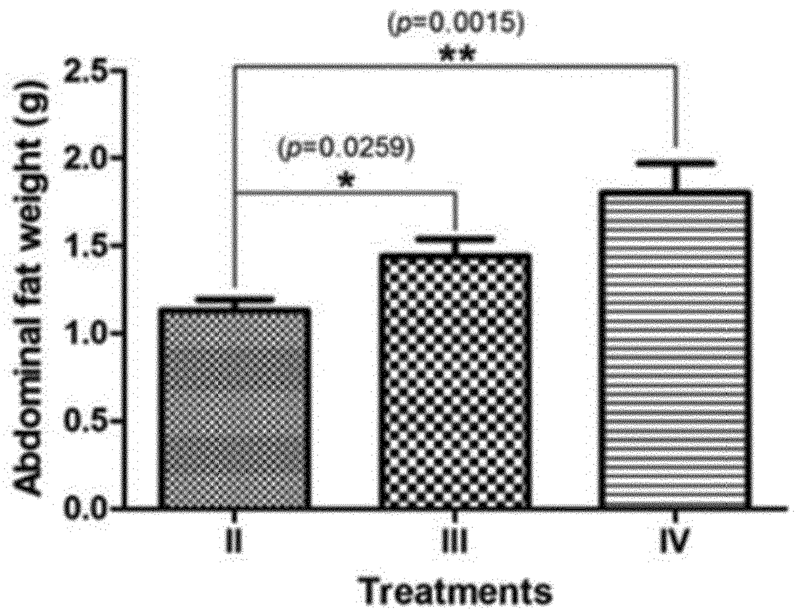
(p=0.0015)
**
(p=0.0259)
*
Abdominal fat weight (g)
2.5
2.0
1.5
1.0
0.5
0.0
II
III
IV
Treatments

FIG. 9

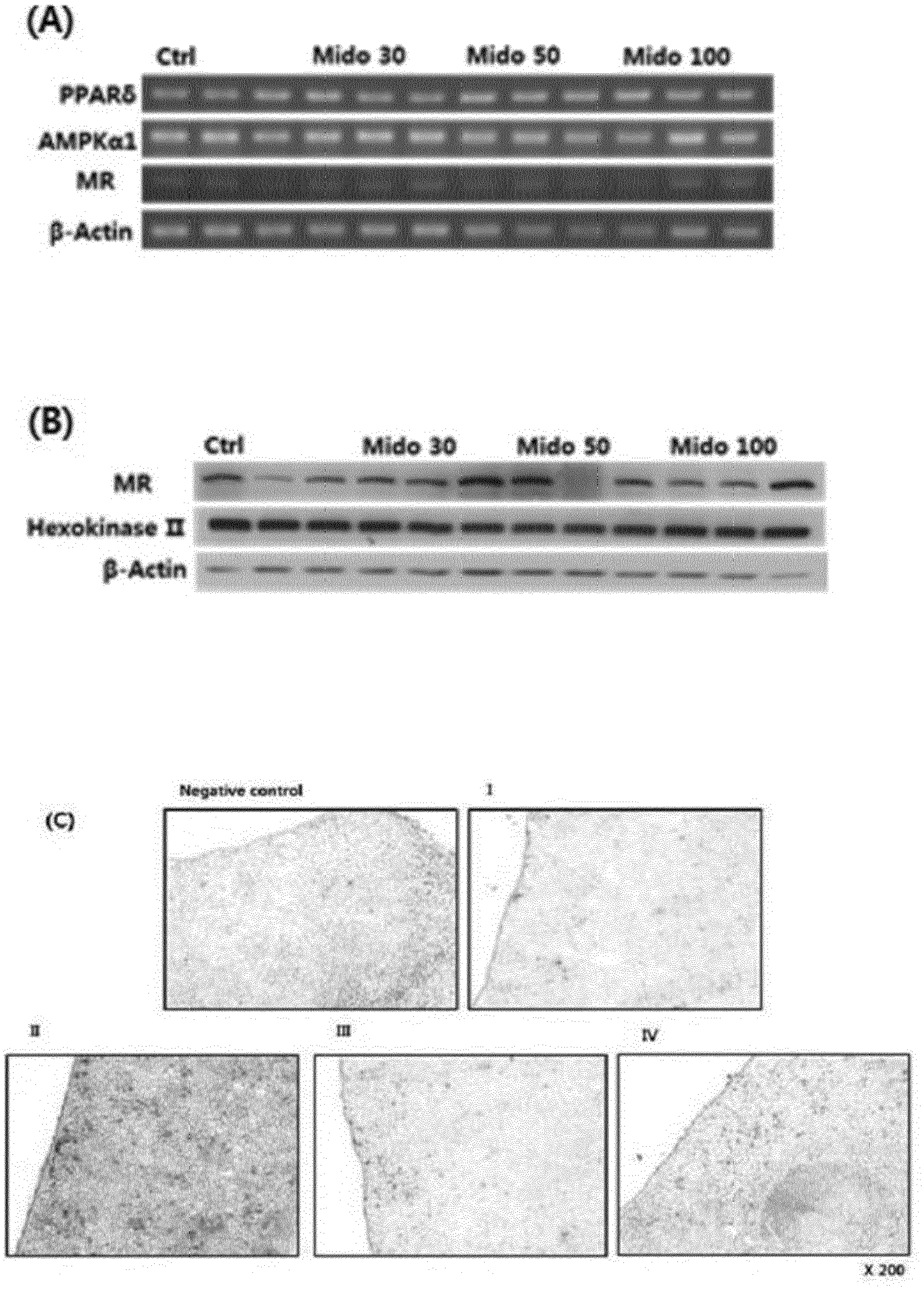
(A)
Ctrl
Mido 30
Mido 50
Mido 100
PPARδ
AMPKα1
MR
β-Actin
(B)
Ctrl
Mido 30
Mido 50
Mido 100
MR
Hexokinase II
β-Actin
(C)
Negative control
I
II
III
IV
X 200

FIG. 10

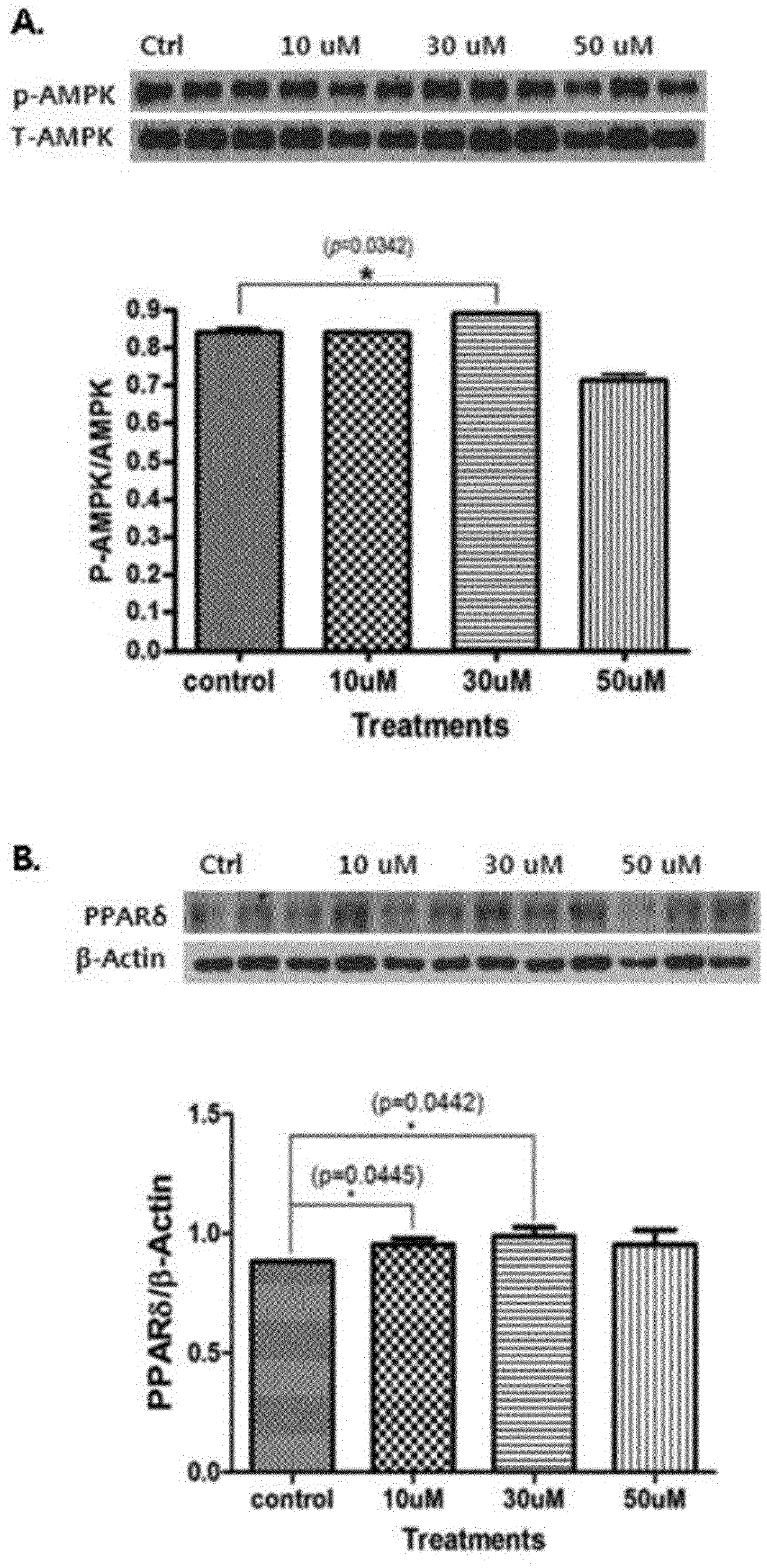
A.
Ctrl
10 uM
30 uM
50 uM
p-AMPK
T-AMPK
(p=0.0342)
*
P-AMPK/AMPK
0.9
0.8
0.7
0.6
0.5
0.4
0.3
0.2
0.1
0.0
control
10uM
30uM
50uM
Treatments
B.
Ctrl
10 uM
30 uM
50 uM
PPARδ
β-Actin
(p=0.0442)
*
(p=0.0445)
*
PPARδ/β-Actin
1.5
1.0
0.5
0.0
control
10uM
30uM
50uM
Treatments

FIG. 11

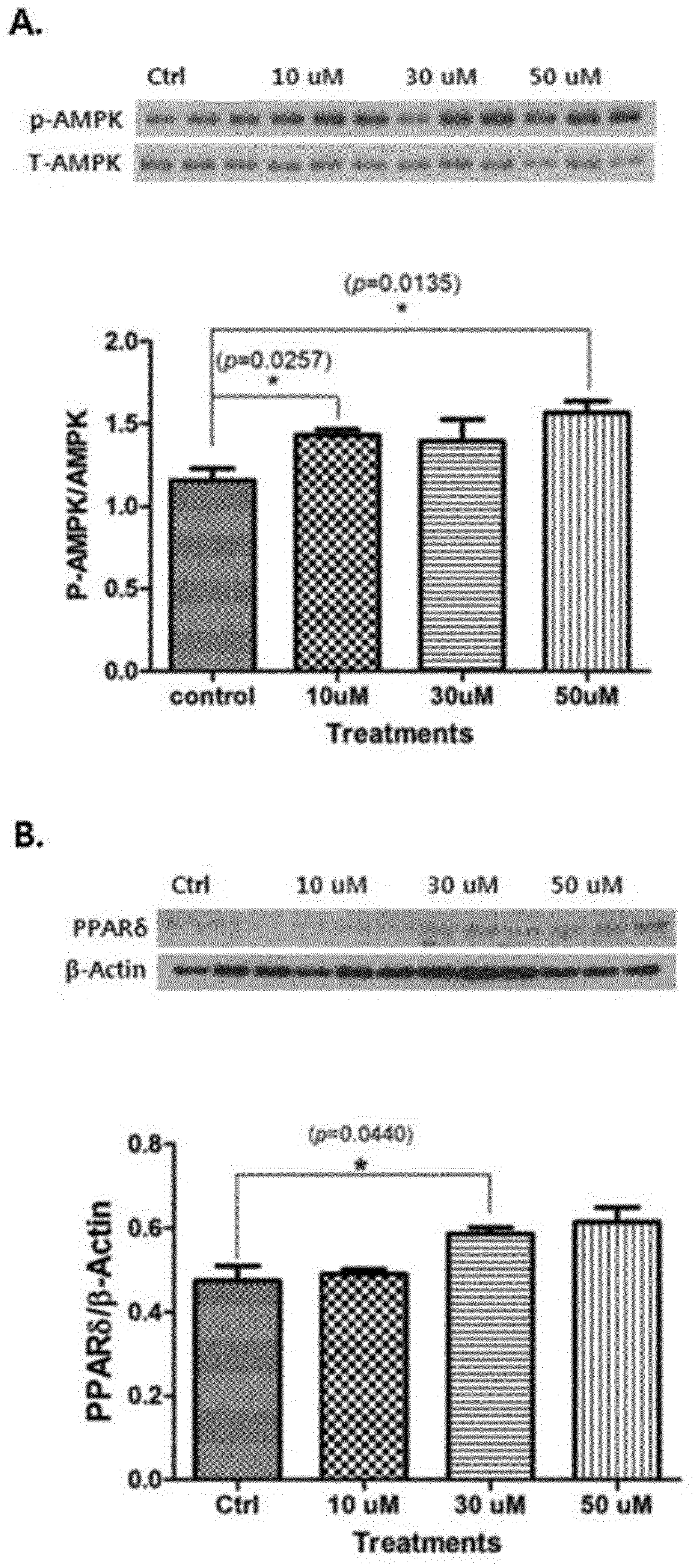
A.
Ctrl
10 uM
30 uM
50 uM
p-AMPK
T-AMPK
(p=0.0135)
*
(p=0.0257)
*
P-AMPK/AMPK
2.0
1.5
1.0
0.5
0.0
control
10uM
30uM
50uM
Treatments
B.
Ctrl
10 uM
30 uM
50 uM
PPARδ
β-Actin
(p=0.0440)
*
PPARδ/β-Actin
0.8
0.6
0.4
0.2
0.0
Ctrl
10 uM
30 uM
50 uM
Treatments

FIG. 12

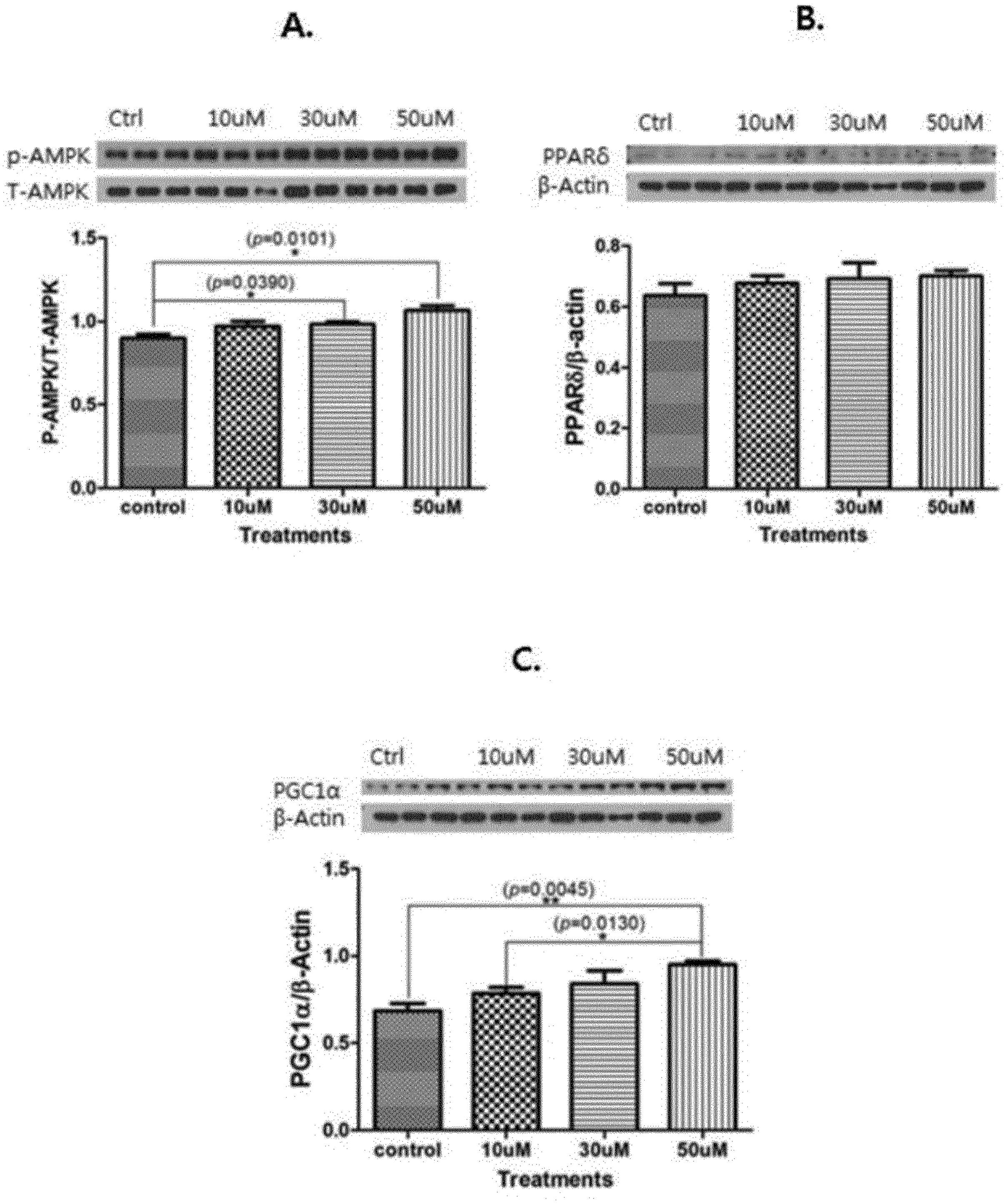
A.
Ctrl
10uM
30uM
50uM
p-AMPK
T-AMPK
P-AMPK/T-AMPK
(p=0.0101)
*
(p=0.0390)
*
1.5
1.0
0.5
0.0
control
10uM
30uM
50uM
Treatments
B.
Ctrl
10uM
30uM
50uM
PPARδ
β-Actin
PPARδ/β-actin
0.8
0.6
0.4
0.2
0.0
control
10uM
30uM
50uM
Treatments
C.
Ctrl
10uM
30uM
50uM
PGC1α
β-Actin
PGC1α/β-Actin
(p=0.0045)
**
(p=0.0130)
*
1.5
1.0
0.5
0.0
control
10uM
30uM
50uM
Treatments

FIG. 13

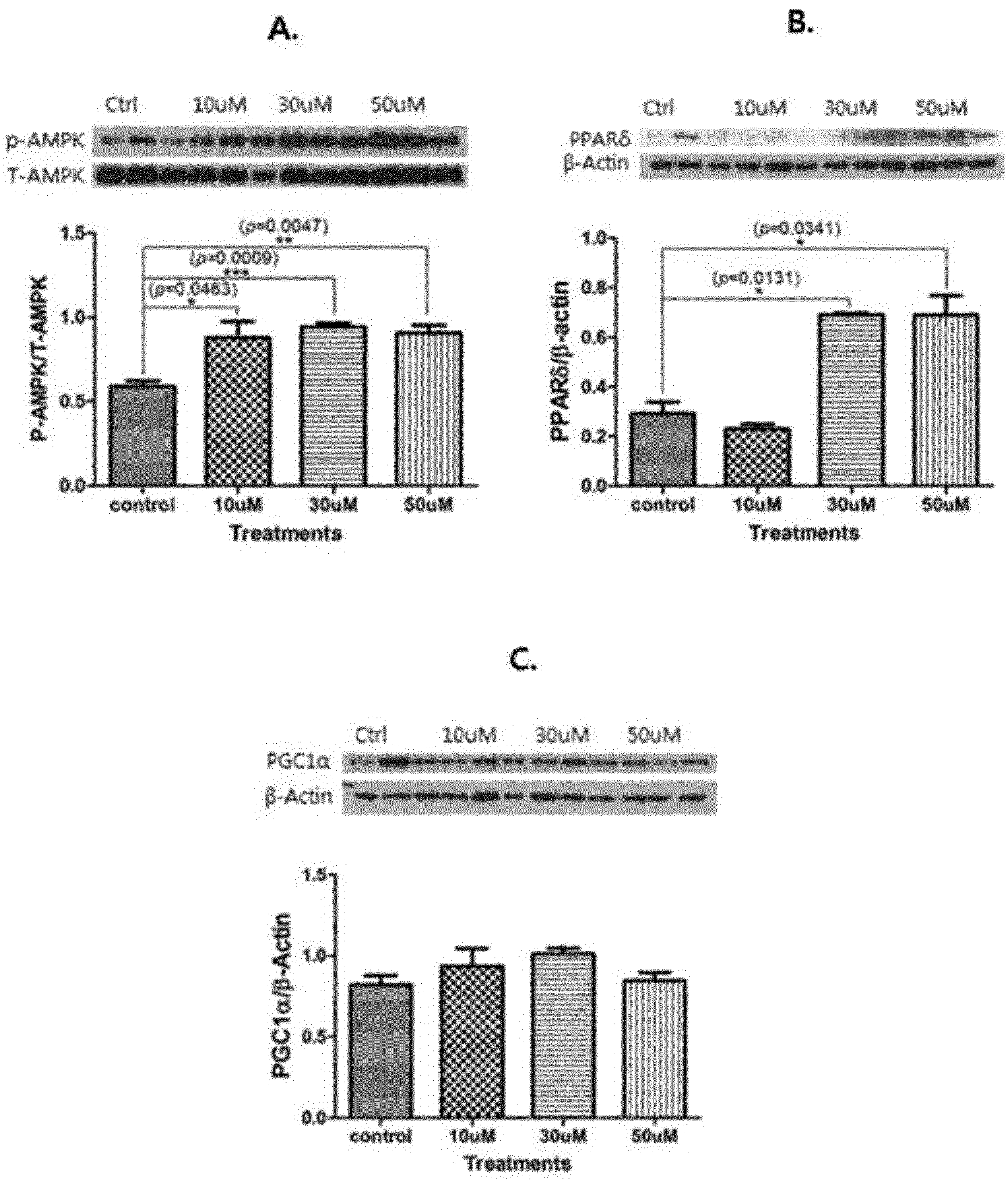
A.
Ctrl
10uM
30uM
50uM
p-AMPK
T-AMPK
(p=0.0047)
**
(p=0.0009)
***
(p=0.0463)
*
P-AMPK/T-AMPK
1.5
1.0
0.5
0.0
control
10uM
30uM
50uM
Treatments
B.
Ctrl
10uM
30uM
50uM
PPARδ
β-Actin
(p=0.0341)
*
(p=0.0131)
*
PPARδ/β-actin
1.0
0.8
0.6
0.4
0.2
0.0
control
10uM
30uM
50uM
Treatments
C.
Ctrl
10uM
30uM
50uM
PGC1α
β-Actin
PGC1α/β-Actin
1.5
1.0
0.5
0.0
control
10uM
30uM
50uM
Treatments

FIG. 14

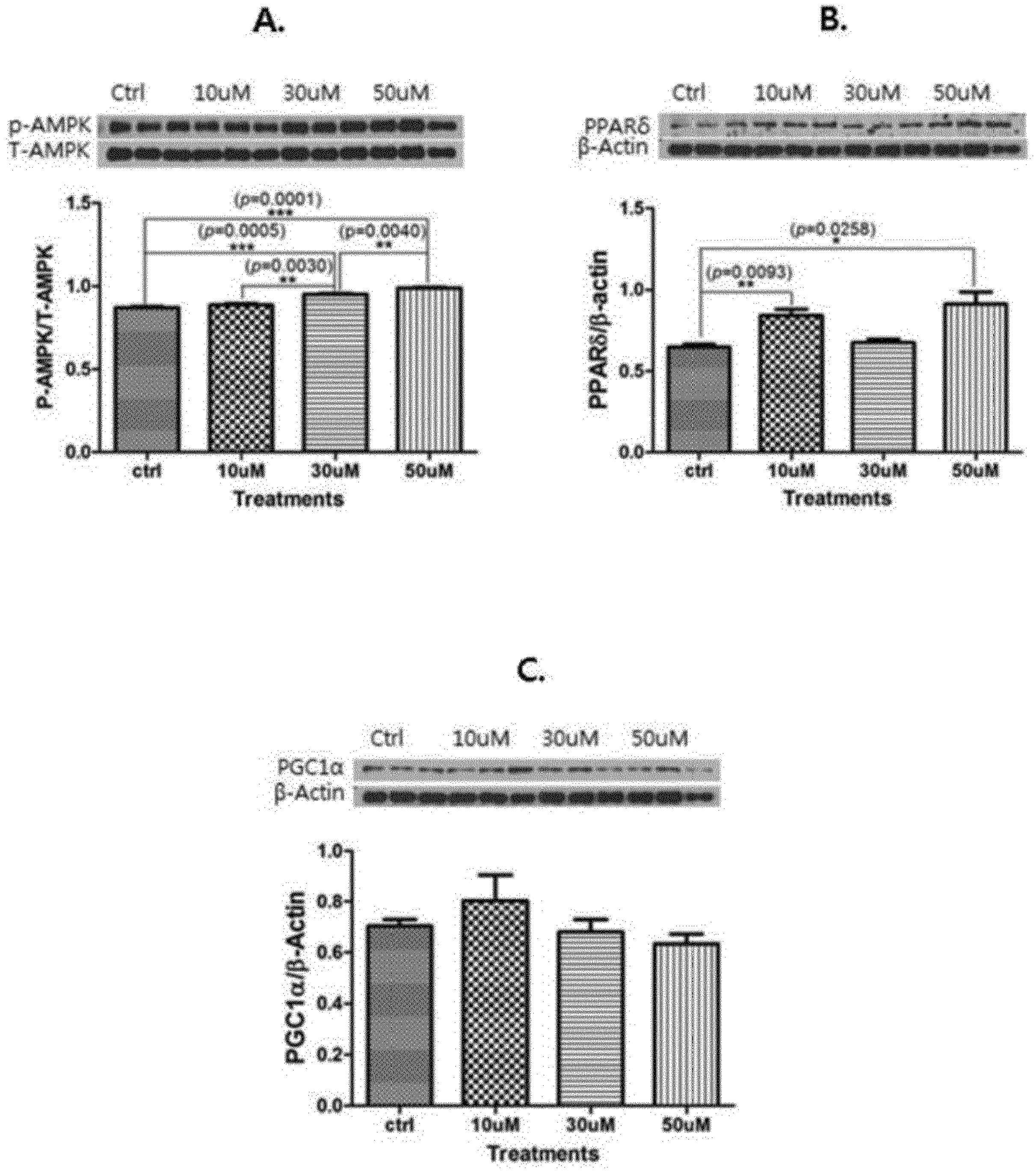
A.
Ctrl
10uM
30uM
50uM
p-AMPK
T-AMPK
P-AMPK/T-AMPK
(p=0.0001)
***
(p=0.0005)
***
(p=0.0040)
**
(p=0.0030)
**
1.5
1.0
0.5
0.0
ctrl
10uM
30uM
50uM
Treatments
B.
Ctrl
10uM
30uM
50uM
PPARδ
β-Actin
PPARδ/β-actin
(p=0.0258)
*
(p=0.0093)
**
1.5
1.0
0.5
0.0
ctrl
10uM
30uM
50uM
Treatments
C.
Ctrl
10uM
30uM
50uM
PGC1α
β-Actin
PGC1α/β-Actin
1.0
0.8
0.6
0.4
0.2
0.0
ctrl
10uM
30uM
50uM
Treatments

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20150136009
- KR 20160122779
- KR 20160122780
- EP 3124019 A
- KR 20130065133 A
- EP 16849065


### Non-patent literature cited in the description


- *Cell,* 2008, vol. 134, 405-415
- **SCHRAGE WG et al.** *J Appl Physiol,* 2004, vol. 97, 1978-1984
- **SCHRAGE WG et al.** *FASEB J,* 2004, vol. 18
- **BRÄNDLE J et al.** *Wiener Klinische Wochenschrift,* 1977, vol. 89, 164-167
- **EISENACH J et al.** *Med Sci Sports and Exercise,* 2004, vol. 36, 157